# EUROPEAN PATENT SPECIFICATION

(11) **EP 1 633 292 B1**
(45) Date of publication and mention of the grant of the patent: **24.02.2010**
(21) Application number: 04735554.0
(22) Date of filing: 01.06.2004
(51) Int. Cl.: A61F 5/441

(54) **AN ADHESIVE FACE PLATE FOR AN OSTOMY APPLIANCE**
EIN SELBSTKLEBENDER KUPPLUNGSRING FÜR EINE OSTOMIEVORRICHTUNG
PLAQUE AVANT ADHESIVE POUR ACCESSOIRE DE STOMIE

(30) Priority: 02.06.2003 DK 200300815; 22.12.2003 DK 200301912
(43) Date of publication of application: 15.03.2006
(73) Proprietor: HOLLISTER INCORPORATED, Libertyville, Illinois 60048-3781 (US)
(72) Inventor: PEDERSEN, Ole, DK-2000 Frederiksberg (DK); WOHLGEMUTH, Jan, DK-2970 Hørsholm (DK); KARLSSON, Michael, Ove, DK-3000 Helsingør (DK)
(74) Representative: Elmeros, Claus
(86) International application number: PCT/DK2004/000378
(87) International publication number: WO 2004/105657

(56) References cited:
- EP-A- 0 255 310
- EP-A- 0 373 795
- EP-A- 0 903 130
- EP-A- 0 988 843
- EP-A- 1 163 892
- WO-A-00/30576
- WO-A-98/55057
- WO-A-03/026541
- GB-A- 1 586 823
- GB-A- 2 290 974
- US-A- 3 897 781
- US-A- 4 232 672
- US-A- 4 826 495
- US-A- 6 071 268
- US-A1- 2003 004 477

## Description

The present invention relates to an adhesive face plate for securing an ostomy bag to the skin of an ostomy patient, said faceplate comprising a flexible plate with a bodyside or proximal surface and a distal surface and comprising a layer of a skin friendly adhesive material and a stoma receiving aperture for receiving a stoma of said patient, generally annular face plate coupling means having an inner and an outer periphery and adapted for fixedly or releasably engaging corresponding bag coupling means of said ostomy bag, said face plate coupling means being secured to said flexible plate with the plane of said face plate coupling means generally parallel to the plane of said flexible plate and generally concentric with said aperture for receiving said stoma, and a filter for sing flatus gas.

"GB 1586823 discloses a face plate of the type in reference having a face plate coupling ring for coupling with a corresponding coupling ring on a pouch A flatus gas passageway is provided extending through the body of the face plate coupling ring from an inner periphery to an outer periphery thereof. A filtering or deodorising agent may be contained in the passageway for deodorising flatus gas flowing through the passageway. Because of the arrangement of the filtering agent in the passageway, the passageway is very prone to being clogged by stomal effluent being built up in front of the filtering agent whereby flatus gas cannot be vented from the ostomy bag."

As the de-odorising filter is relatively expensive, it is desirable to re-use the filter in connection with several consecutive disposable ostomy bags by attaching the filter to the face plate instead of to the ostomy bag such that the filter is not disposed of together with the full ostomy bag.

US patent No. 4,232,672 discloses a face plate of the type in reference where a face plate coupling means in the form of a ring is provided with a lateral projection comprising a filter chamber containing filters communicating with two flatus gas passages extending through the coupling ring to the interior periphery thereof. Manually operable valve means allow flatus gas to flow through said passages and through the filters. This is a relatively complicated and expensive structure requiring manual action to vent the flatus gas. The dimension of the coupling ring perpendicular to the plane thereof is also relatively large so as to accommodate the venting mechanism, and thus the face plate is relatively bulky, which is undesirable.

US Patent No; 4,826,495 also discloses a face plate of the type in reference having a relatively thick retainer plate having an annular projection containing an annular duct communicating with the interior of an ostomy bag attached to the coupling ring through flatus gas inlet apertures in said annular projection.
The annular duct communicates with a filter arranged in a recess provided in the body of the retainer plate. This is also a relatively expensive and complicated structure that also is relatively bulky.

It is a main object of the invention to provide an adhesive face plate with an integrated flatus gas de-odorising filter that is relatively simple to manufacture, is relatively inexpensive and has a maximum dimension perpendicular to the plane of the coupling ring that is relatively small such that the entire face plate is not unduly bulky (low profile).

According to the invention, this object is obtained by providing one or more flatus gas passages extending between said face plate coupling means and said flexible plate from said inner periphery to said outer periphery and through said filter.

Hereby, the coupling means is not affected, and the flatus gas passages may be provided without requiring expensive and bulky interior passageways in the coupling means.

In the currently preferred embodiment of the invention, the face plate coupling means and the bag coupling means are mutually matching coupling rings adapted for releasably engaging one another.

Alternatively, the face plate coupling means and the bag coupling means are mutually matching surfaces adapted for being releasably or fixedly adhered to one another by means of an adhesive or fixedly adhered to one another by means a heat seal seam.

According to the invention, the face plate may further comprise a compartment having an exterior portion located outside said outer periphery and an interior portion located between said coupling means and said flexible plate, a flatus gas outlet being provided in a wall of said exterior portion, said compartment forming part of said flatus gas passage, said filter being located within said compartment such that said flatus gas outlet is obstructed by said filter.

Hereby, a flatus gas filter housing is provided without requiring bulky and expensive modifications of the coupling ring.

In the currently preferred embodiment of a face plate according to the invention said exterior portion of said compartment is defined by a first film strip of plastic material, preferably adapted to define a pouch.

Hereby, a particularly inexpensive, low profile and easily manufactured filter housing having small space requirements is provided.

In another aspect, the present invention relates to an adhesive face plate for securing an ostomy bag to the skin of an ostomy patient, said faceplate comprising a flexible plate with a bodyside or proximal surface and a distal surface and comprising a layer of a skin friendly adhesive material and a stoma receiving aperture for receiving a stoma of said patient, generally annular face plate coupling means having an inner and an outer periphery and adapted for fixedly or releasably engaging corresponding bag coupling means of said ostomy bag, said face plate coupling means being secured to said flexible plate with the plane of said face plate coupling means generally parallel to the plane of said flexible plate and generally concentric with said aperture for receiving said stoma, a filter for de-odorising flatus gas, a flatus gas venting compartment in the form of a pouch or bag made of a first film of plastic material and extending outward beyond said outer periphery, said first film being provided with a flatus gas outlet aperture, and a flatus gas passage extending from said inner periphery into the interior of said venting pouch and through both said filter and said flatus gas outlet aperture, said filter being arranged such that substantially all said flatus gas vented through said flatus gas aperture flows through said filter.

Hereby, a simple, low profile and inexpensive face plate with integrated filter is provided.

The flatus gas passage may extend between said coupling means and said flexible plate or it may extend at least partly through a conduit extending through a portion of said coupling means from a conduit inlet to a conduit outlet.

In the latter case one edge of said pouch may by attached to a body side or proximal surface of said coupling means facing said flexible plate and the opposed edge of said pouch may be attached to a distal surface of said coupling means facing away from said flexible plate, said conduit outlet being located between said proximal and distal surfaces such that flatus gas exiting from said conduit enters said pouch.

In a yet further aspect, the present invention relates to an ostomy appliance for receiving human stomal discharge comprising in combination an adhesive face plate according to the invention and an ostomy bag for receiving human stomal discharge releasably or fixedly attached to said face plate.

In the currently preferred embodiment of the invention obstructing means for hindering or preventing stomal fluids and liquids entering said flatus gas passage is provided adjacent the inner periphery of the face plate coupling means and preferably the obstructing means comprises an elongate body of a gas permeable, preferably hydrophobic material such as open cell foam or non-woven material, said body having an arcuate, preferably closed loop and preferably annular shape and being located between said face plate coupling means and said stoma receiving aperture.

Preferably, the elongate body is attached to the ostomy bag and/or to the bag coupling means such that removal of the bag entails removal of the elongate body such that cleaning of the face plate when replacing the ostomy bag is reduced or completely eliminated.

In the following, the invention will be explained more in detail with reference to various embodiments thereof shown, solely by way of example, in the accompanying drawings, where
Fig. 1 is a diagrammatic top plan view of a first embodiment of a face plate according to the invention,
Fig. 2 is a cross section of the embodiment of Fig. 1 taken along line A-A,
Fig. 3 is a diagrammatic top plan view of a second embodiment of a face plate according to the invention,
Fig. 4 is a cross section of the embodiment of Fig. 2 taken along line B-B,
Fig. 4A is a cross sectional view corresponding to Fig. 4 of the face plate of Figs. 3-4 releasably connected to an ostomy bag for collecting human discharge,
Fig. 5 is a diagrammatic top plan view of a third embodiment of a face plate according to the invention,
Fig. 6 is a cross section of the embodiment of Fig. 5 taken along line C-C,
Fig. 7 is a diagrammatic top plan view of a fourth embodiment of a face plate according to the invention,
Fig. 8 is a cross section of the embodiment of Fig. 7 taken along line D-D,
Fig. 9 is a diagrammatic top plan view of a fifth embodiment of a face plate according to the invention,
Fig. 10 is a cross section of the embodiment of Fig. 9 taken along line E-E,
Fig. 11 is a diagrammatic top plan view of a sixth embodiment of a face plate according to the invention,
Fig. 12 is a cross section of the embodiment of Fig. 11 taken along line F-F,
Fig. 13 is a diagrammatic top plan view of a seventh embodiment of a face plate according to the invention,
Fig. 14 is a cross section of the embodiment of Fig. 13 taken along line G-G,
Fig. 15 is a diagrammatic top plan view of an eighth embodiment of a face plate according to the invention,
Fig. 16 is a cross section of the embodiment of Fig. 15 taken along line H-H,
Fig. 17 is a diagrammatic top plan view of a ninth embodiment of a face plate according to the invention,
Fig. 18 is a cross section of the embodiment of Fig. 17 taken along line I-I,
Fig. 19 is an enlarged scale view of the cross section in Fig. 18 through the coupling ring,
Fig. 20 is an enlarged scale broken away elevational view seen in the direction of the arrow R in Fig. 17,
Fig. 21 is a cross sectional view corresponding to Fig. 4A of a further embodiment of an ostomy device according to the invention with an inner disposable pouch within an outer pouch,
Fig. 22 is a cross sectional, broken-away view of the top portion of a further embodiment of an ostomy device according to the invention,
Fig. 23 is a cross sectional broken away view of the top portion of a further embodiment of an ostomy device according to the invention,
Fig. 24 is a cross sectional broken away view of the top portion of a further embodiment of an ostomy device according to the invention with an adhesive releasable attachment of the ostomy pouch to the face plate,
Fig. 25 is a cross sectional broken away view of the top portion of a further embodiment of an ostomy device according to the invention where the ostomy pouch is permanently attached to the face plate, a so-called one-piece ostomy appliance,
Fig. 26 is a cross sectional broken away view of the top portion of a further embodiment of an ostomy device according to the invention where the face plate is convex for being adapted to a depression in the peristomal skin surface of a user of the ostomy device,
Fig. 27 is a cross sectional broken away view of the top portion of a further embodiment of an ostomy device according to the invention with a convex face plate,
Fig. 28 is a cross sectional broken away view of the top portion of a further embodiment of an ostomy device according to the invention with a convex face plate, and
Figs. 29 - 31 are cross-sectional broken away views of three embodiments of a two-piece ostomy appliance according to the invention with means to reduce the cleaning of the face plate when replacing the ostomy bag of the appliance.

In the drawings, similar elements of the different embodiments are referred to by the same reference numerals.

All the embodiments of a faceplate according to the invention are referred to generally by the numeral 1 and comprise a layer 2 of a pressure-sensitive skin friendly adhesive.

The adhesive layer 2 may be formed of any suitable pressure-sensitive adhesive commonly used for securing the faceplates of ostomy appliances to the peristomal skin surfaces of a wearer. For example, a hypoallergenic medical-grade acrylic adhesive may be used. However, it is preferable that the adhesive layer be formed of a soft, skin friendly hydrocolloid-containing adhesive material that is capable of absorbing moisture and has both wet and dry tack. Such a material is commonly referred to as a skin barrier composition and typically comprises a continuous elastomeric adhesive phase having hydrocolloid particles dispersed throughout the continuous phase. Initial tack, usually referred to as "dry tack," is provided by the continuous phase but, because such a composition is occlusive or non-breathable, adherence to the skin would be disrupted by perspiration and by liquid stomal discharge if it were not for the dispersed hydrocolloids which absorb fluids and thereby maintain and possibly enhance adhesive attachment to the skin. U.S. patent No. 4,551,490 and other references disclose that suitable water-absorbing and swellable hydrocolloid gums may include sodium carboxymethylcellulose, pectin, gelatin, guar gum, locust bean gum, and the like. The elastomers used in the continuous phase may be polyisobutylene, natural rubber, silicone rubber, acrylonitrile rubber and other elastomers known in the art to have similar properties.

A silicone treated release sheet 3 of plastic material is applied to the body side or proximal surface of the adhesive layer 2 for protecting the adhesive layer 2 until the faceplate is to be applied to the skin of a wearer thereof.

A carrier sheet 4 of heat sealable plastic material is attached to the distal surface of the adhesive layer 2. An aperture 5 for receiving a stoma of a wearer of the face plate is provided in the layer 2 and in the sheets 3 and 4.

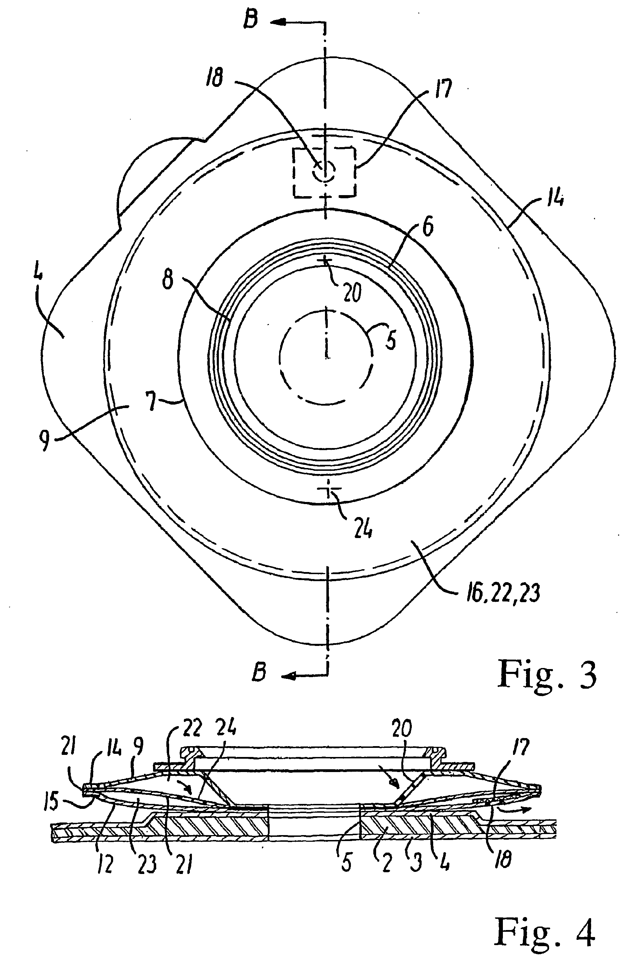

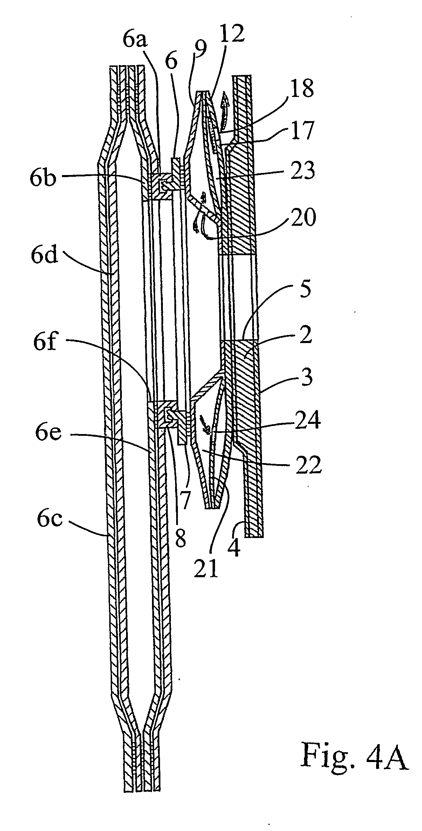

A coupling ring 6 of a flexible plastic material and having an outer periphery 7 and an inner periphery 8 is adapted for engaging a corresponding coupling ring 6a heat sealed on an ostomy bag 6b covered by a comfort layer 6c of non-woven material (Fig. 4A) for securing the bag to the wearer such that stomal material may be collected in the bag when the faceplate 1 with the bag secured to the faceplate is adhered to the peristomal skin of the wearer.

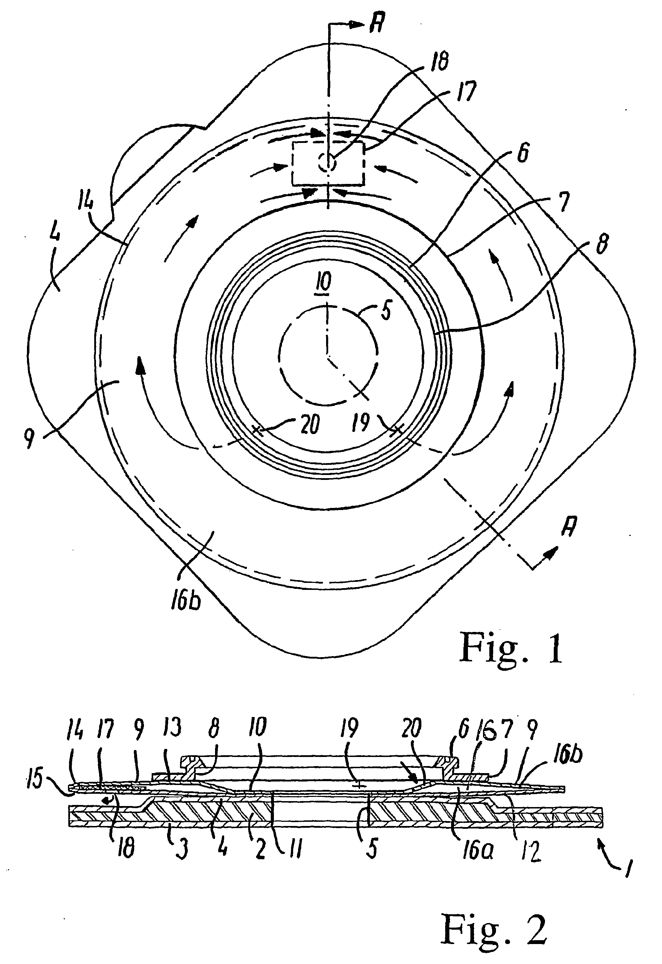

Referring now to Figs 1-2, the coupling ring 6 is secured to the adhesive layer 2 by means of a first annular strip 9 of film of a heat sealable plastic material having one edge 10 attached by heat sealing to one edge 11 of a second annular strip 12 of film of a. heat sealable plastic material which edge 11 in turn is attached to the carrier sheet 4 by heat sealing. The coupling ring 6 is attached to the film strip 9 at 13 by heat sealing whereby the coupling ring 6 is flexibly attached to the adhesive layer 2.

Edges 14 and 15 of the strips 9 and 12, respectively, spaced outwardly from the edges 10 and 11; respectively, are heat sealed to one another such that an annular compartment or chamber 16 is formed having an interior portion 16a located between the coupling ring 6 and the adhesive layer 2 and an exterior portion 16b in the form of a pouch located outside the exterior periphery 7 of the coupling ring 6. The film strips 9 and 12 may be integral such that the heat sealing of edges 14 and 15 may be avoided.

A flatus gas de-odorising filter 17 is arranged in the exterior portion 16b of the compartment 16 and is adhered or heat sealed to film strip 12 such that it covers a flatus gas outlet aperture 18 provided in film strip 12. Two (or more) spaced flatus gas inlet apertures 19 and 20 are provided in the portion of the film strip 9 located inside the inner periphery 8 of the coupling ring 6. The inlet apertures 19 and 20 are located remote from the outlet gas aperture 18. The inlet apertures 19 and 20 are made by cutting a cross-shaped incision in the film strip 9.

In use, the release sheet 3 is removed from the adhesive layer 2, and the proximal surface of the layer 2 is applied to the peristomal skin of a wearer such that the stoma is received in the aperture 5. A collecting ostomy bag is then attached to the faceplate 1 by engaging the coupling ring of the bag with the matching coupling ring 13.

Stomal material consisting of solid material, liquid and flatus gas is then discharged from the stoma into the collecting or ostomy bag. The solid material and liquid falls down into the bag while the flatus gas is led out of the bag through the flatus gas passages defined by the inlet apertures 19 and 20, the interior compartment portion 16a and the exterior compartment portion 16b, the filter 17 and the flatus gas outlet 18. The flow of the flatus gas is indicated by means of arrows.

The filter 17 is of the type disclosed in US Patent No. 6,506,184.
However, any other suitable type of deodorising filter may be used.

In this embodiment and the embodiments shown in Figs. 3-12 and Figs. 17-20, the coupling ring 6 is flexibly attached to the flexible plate 2, 3, 4 with the advantages inherent in this design as explained and disclosed in co-owned US patents No. 4,419,100 and 5,730,735.

Referring now to Figs. 3-4A, this embodiment is identical to the embodiment of Figs. 1-2 except for the provision of an intermediate strip 21 of a film of plastic material attached by heat sealing or adhesion to the film strips 9 and 12 such that the compartment 16 is subdivided into two annular compartments 22 and 23, the filter 17 being located in compartment 23. An aperture 24 is provided in the film strip 21 at a location opposite the inlet aperture 20 such that the flow of flatus gas indicated by the arrow is forced to flow through the labyrinth defined by the compartment 22, the aperture 24 and the compartment 23 before reaching the filter 17 and exiting through the flatus gas outlet 18. If any stomal liquid or solid material enters the compartment 16 through the inlet aperture 20 the labyrinth created by interposing the film strip 21 will create a larger distance between the inlets 19 and 20 and the filter 17 such that the probability of any such liquid or solid material clogging the filter is reduced.

The ostomy bag 6b is a conventional bag comprising two film sheets 6d and 6e heat sealed to one another along the edges thereof to form a bag having an aperture 6f for receiving stomal discharge. The comfort layer 6c is heat sealed to the film walls 6d and 6e along the outer edge thereof.

The film 21 may be substituted by a layer of non-woven material or other material that is permeable to flatus gasses, but hinders flow of liquid through the layer. In such case it is not necessary to provide a flatus gas aperture 24.

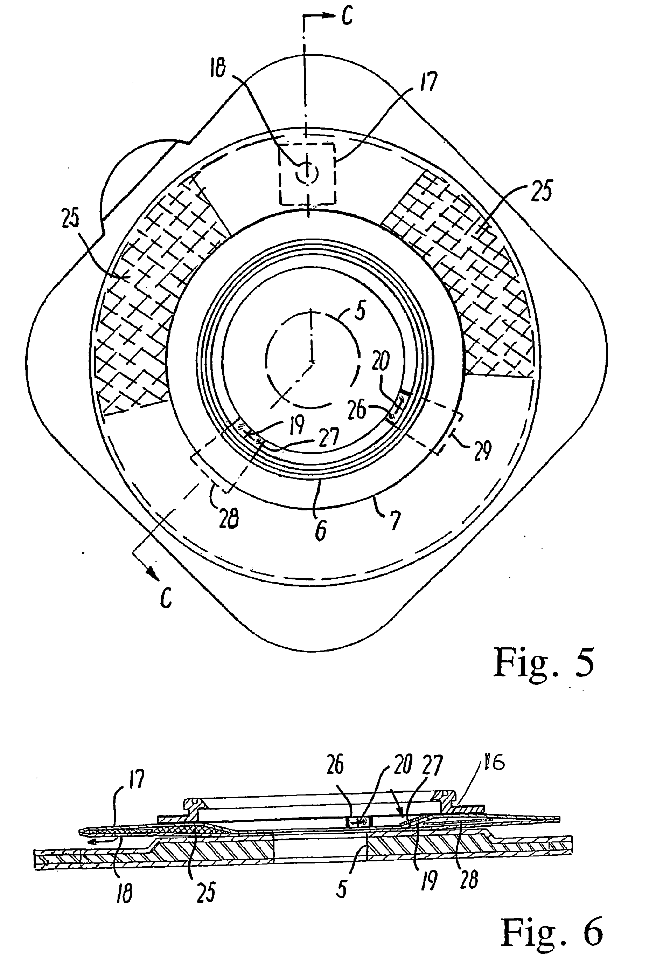

Referring now to Figs. 5-6, this embodiment is the same as the one in Figs. 1-2 with the following exceptions:
- open cell foam material 25 or other suitable porous material is interposed in the annular compartment 16 between the inlets 19 and 20 and the filter 17, the foam material preferably being hydrophobic.
- strips 26 and 27 of a gas permeable, liquid impermeable material are adhered to the film strip 9 around the apertures 19 and 20, respectively, such that the strips 26 and 27 cover said apertures, and
- strips 28 and 29 of non-woven material are arranged in the compartments 16 adjacent the inlet apertures 19 and 20, respectively, so as to function for ensuring that there is a space between the film strips 9 and 12 adjacent the inlet openings 19 and 20 even though the film strips 9 and 12 have a tendency to cling or adhere to one another because of moisture or other factors.

The open cell foam material 25 fills out a cross section of the chamber 16 and thus prevents any liquid or solid material entering the chamber 16 through the inlet openings from reaching the filter 17 while still allowing flatus gas to flow through the chamber 16 to the filter.

The strips 26 and 27 of breathable material perform the same function of allowing flatus gas to pass, but at the same time preventing liquid or solid material from entering the compartment 16.

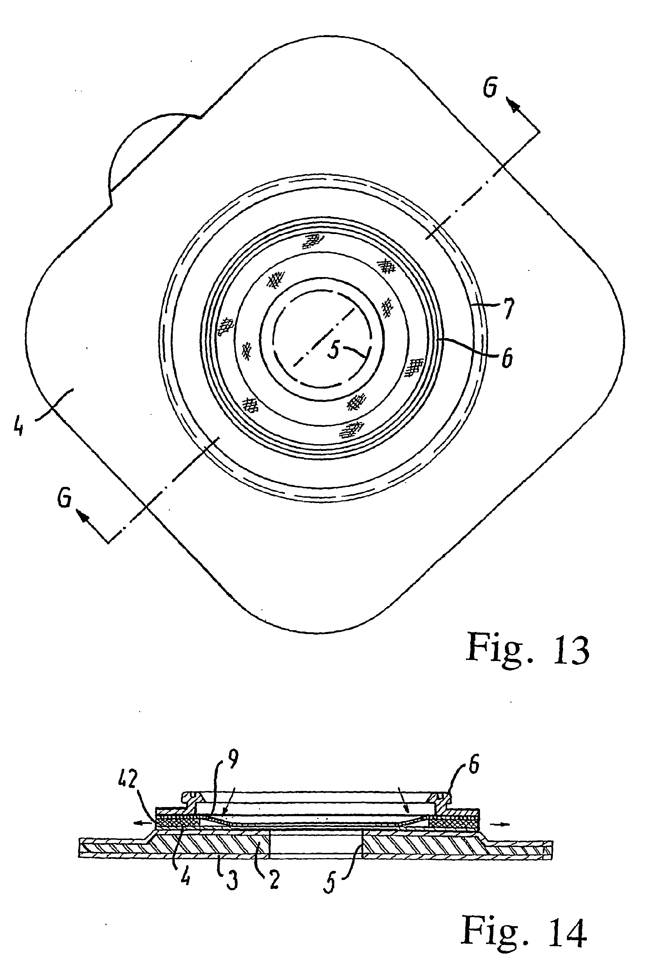

The open cell foam material 25, the gas permeable, liquid impermeable film strips 26 and 27 and the spacing members 28 and 29 of non-woven material may be applied together or individually to all the other embodiments except the embodiment shown in Figs. 13-14. The strips of non-woven material may extend along the entire circumference of the coupling ring.

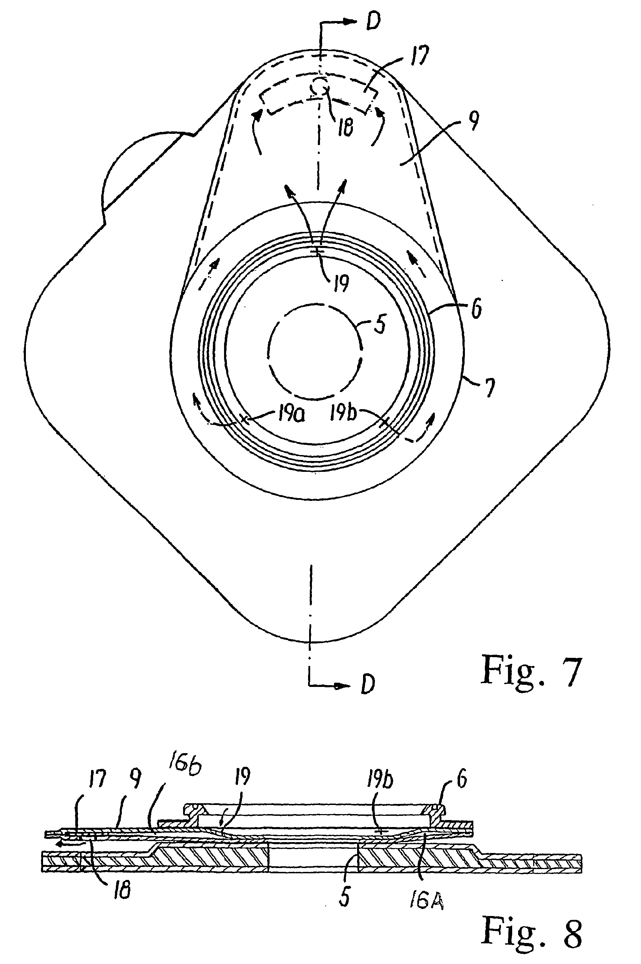

Referring now to Figs. 7-8, this embodiment is very similar to the embodiment in Fig. 1-2 except that the exterior portion or pouch 16b of the compartment does not extend around the entire outer periphery of the coupling ring 6, but only extends along a portion of said periphery. The inlet aperture 19 is shown close to the filter 17, but alternative apertures 19a and 19b could also be arranged opposite the filter relative to the coupling ring as in the Figs. 1-2 embodiment. The flow of flatus gas in connection with the alternative apertures 19a and 19b is indicated with dotted line arrows.

The filter 17 is shown arcuate with the arcuate edges being impermeable to gas such that the flow of flatus gas into the filter takes place through the opposed ends of the filter. The filter 17 could also in this case be square or rectangular as in the Figs. 1-2 embodiment.

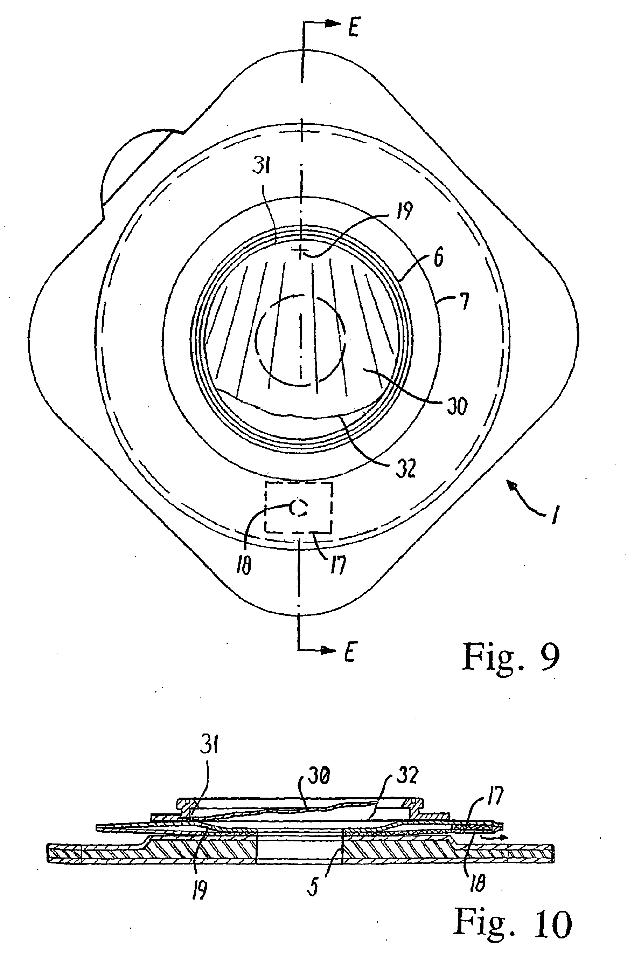

Referring now to Figs. 9-10, this embodiment is identical to the Figs. 1-2 embodiment except for the addition of a curtain 30 made of a patch or strip of film of a plastic material having one edge 31 attached by heat sealing to the coupling ring 6 along part of the circumference hereof and the opposed edge 32 free.

The curtain 30 will tend to prevent or hinder stomal liquid or solids from entering the flatus gas inlet 19 when the face plate 1 with a pouch attached is applied to the patient such that the curtain 30 extends downwards when the patient is standing. The stomal material will fall into the pouch through the aperture defined by the free edge 32 and the flexible face plate 1.

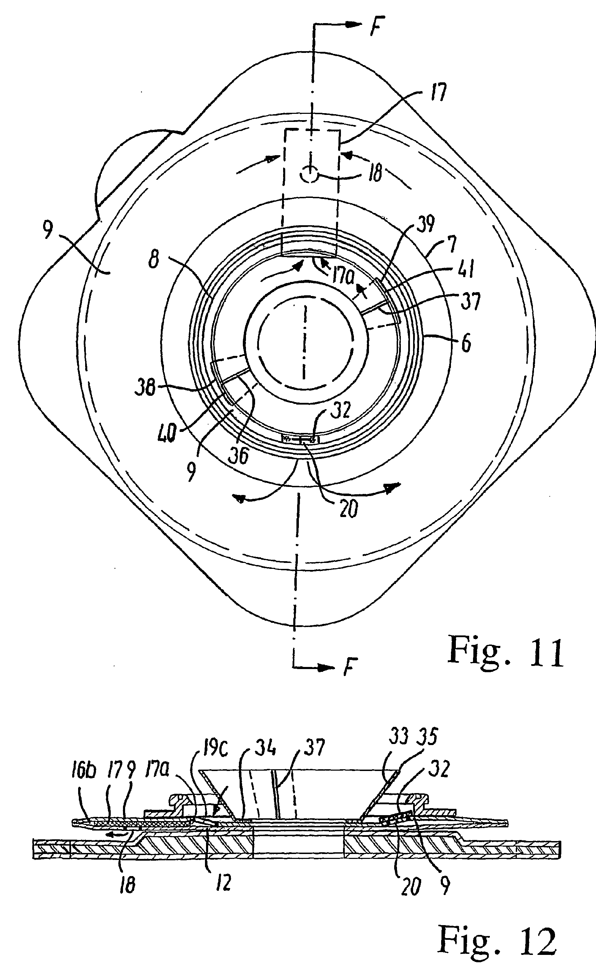

This curtain may be applied to all the other embodiments shown in the drawings except the Figs. 11-12 embodiment.

Referring now to Figs. 11-12, in this embodiment, the filter 17 extends from the exterior compartment portion or pouch 16b between the coupling ring 6 and the flexible plate 2, 3, 4 beyond the inner periphery 8 of the coupling ring through an aperture 19c in the film strip 9 to an edge 17a located within said inner periphery.

A second gas inlet aperture 20 is located opposite the aperture 19c and is covered by a patch or strip of 32 of a gas permeable, liquid impermeable film material. The aperture 19c may also be covered by such a patch of gas permeable, liquid impermeable material.

A strip 33 of a film of plastic material has one edge 34 heat sealed to the film strip 9 while the opposed edge 35 is free such that an annular collar is formed within the inner periphery 8. The film strip 33 is provided with two transverse slits 36 and 37 and further strips 38 and 39, respectively, of a film of a plastic material are heat sealed at 40 and 41, respectively, to the strip 33 on one side of the slits 36 and 37, respectively.

Hereby, the collar 33 may be deformed outwards by pressure from stomal material so to cover the apertures 19c and 20 because of the deformability afforded by the slits 36 and 37 while the overlap of the strips 38 and 39 prevents or hinders that stomal material can be pressed through the slits 36 and 37, respectively.

This collar 33 may be applied to all the other embodiments shown in the drawings except the Figs. 9-10 embodiment.

Referring now to Figs. 13-14, in this embodiment an annular filter 42 is arranged between the coupling ring 6 and the carrier sheet 4 and is attached to the film strip 9 and the carrier sheet 4 either by heat sealing or by means of an adhesive. The annular film strip 9 is made of a heat sealable, gas permeable, liquid impermeable film. The construction of the filter 42 is such that it has sufficient shear strength to ensure that the coupling ring 6 is securely fastened to the carrier sheet 4 and thereby to the adhesive layer 2.

The flatus gas may flow out at all points along the coupling ring 6 whereby a particularly effective venting of the flatus gas is achieved. An annular filter pouch such as pouch 9, 12 in Figs 1-4A with a flatus escape aperture 18 may be arranged around annular filter 42 for preventing water from blocking filter 42 if the user of the ostomy device showers.

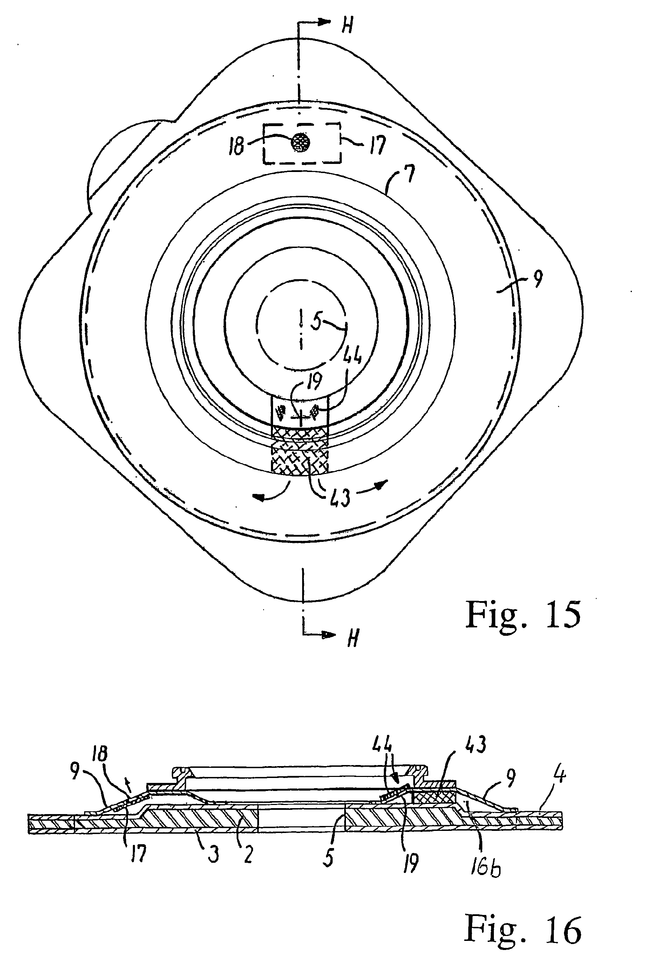

Referring now to Figs. 15-16, in this embodiment the film strip 12 has been eliminated and the edge 14 of the film strip 9 is heat sealed to the flexible plate 2, 3, 4 such that the exterior portion 16b of the compartment is defined by the film strip 9 and the carrier sheet 4.

The flatus gas inlet aperture 19 is covered by a patch of gas permeable, liquid impermeable plastic film, and a spacer 43 in the form of a strip of open cell foam material is arranged adjacent the aperture 19.

The de-odorising filter 17 is adhered to the film strip 9 around a flatus gas outlet aperture 18 provided in the film strip 9.

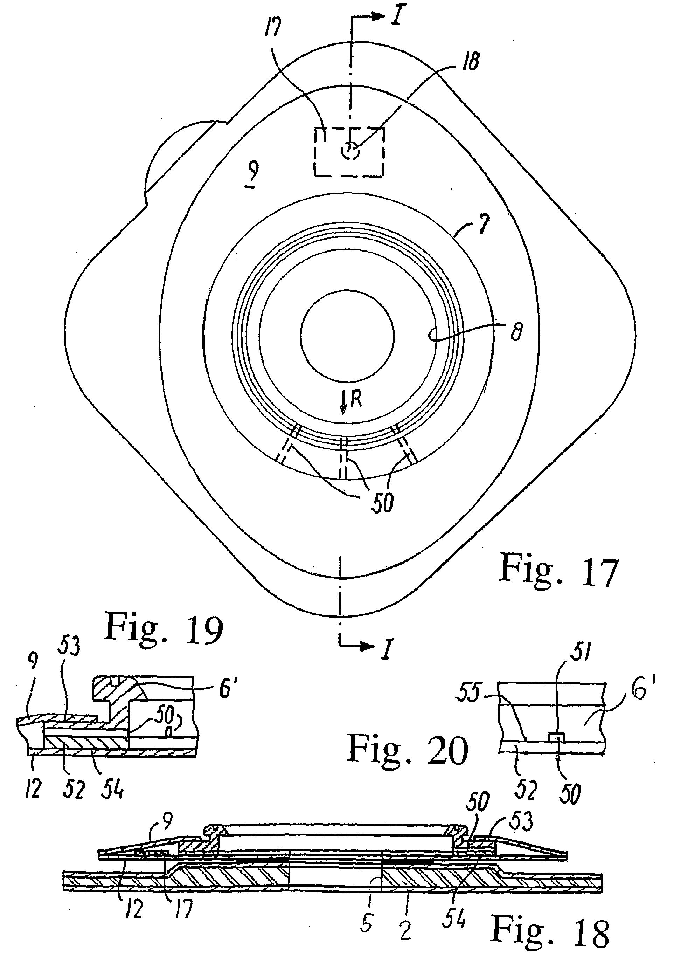

Referring now to Figs. 17-20, an alternative embodiment is shown, wherein the pouch is located in a different manner. The film strip 9 defining the pouch together with film strip 12 has one edge attached to the proximal surface 53 of the coupling ring 6' while the film strip 12 is attached to the distal surface 54 of the coupling ring 6' and to the carrier sheet 4. The flatus gas passage comprises one or more conduits 50 extending through the coupling ring from the interior periphery 8 to the outer periphery 7 such that the outlet of the or each conduit is located between the distal and proximal surfaces so that the gas passage extends between said edges of the film strip defining the pouch.

The conduit 50 may be provided without greatly increasing the dimension of the coupling ring perpendicular to the plane thereof. One way of providing said conduit 50 is illustrated in Fig 20. A channel 51 is formed in the distal surface 55 of a coupling ring very similar to coupling ring 6 shown in Figs. 1-16. A ring 52 is thereafter adhered to said distal surface 55 whereby the coupling ring 6' provided with the conduit 50 is formed.

One advantage of this alternative embodiment is that the coupling ring 6' with attached pouch 9, 12 may be manufactured separately and subsequently attached to the carrier sheet 4 by heat sealing the edge portion of film strip 12 to the carrier sheet.

The shape of the pouch 9, 12 is oval so as to save material for the film strip 9 and 12 and still have sufficient space for any spacing means adjacent the conduits 50 and for the filter 17.

It should be noted that alternatively the flatus gas passage may be provided in the adhesive layer 2 of the flexible plate 2, 3, 4. An elongate filter may also be embedded in the adhesive layer 2 extending from the stoma receiving aperture 5 to the outer periphery of the adhesive layer 2.

This gas passage in the adhesive layer may also be a groove provided in the surface of the adhesive layer 2 covered by the carrier sheet 3, the groove extending from the stoma receiving aperture 5 to an aperture in the carrier sheet 3, The de-odorising filter may be attached to the carrier sheet such that the flatus gas vented through the groove and the aperture is constrained to flow through the filter.

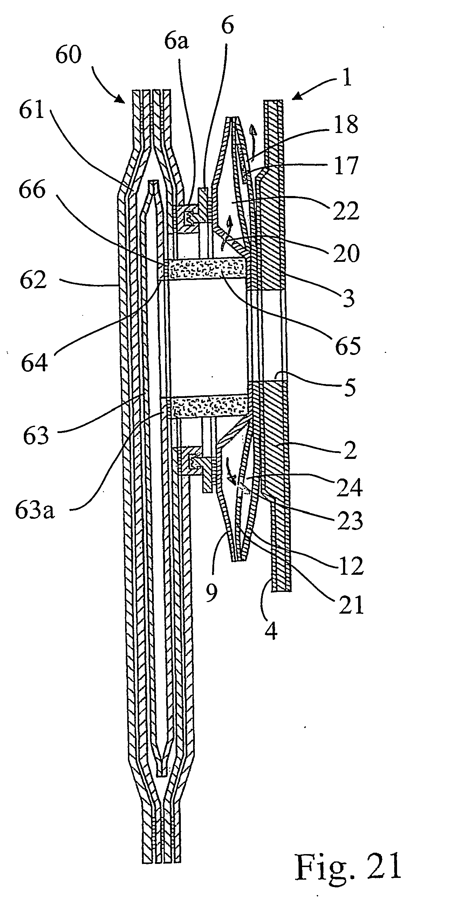

Referring now to Fig. 21, a face plate 1 identical to the one shown in Figs. 3-4A, except that there are a number of apertures 20 distributed around the upper half circle of the film 9, is attached to an ostomy bag referred to generally by the numeral 60 and comprising an outer bag 61 of a liquid and gas impermeable plastic sheet material covered by a comfort layer 62 of a non-woven material.

An inner bag 63 made of plastic sheet material that is biodegradable and/or soluble under certain circumstances is arranged inside the outer bag 61 and is provided with an aperture 64 for receiving stomal discharge. This inner bag 63 is intended for being separated from the outer bag and disposed of by being flushed down a toilet when full of stomal discharge.

So as to avoid that liquid and/or semi-solids from the stomal discharge from entering the filter pouch 9, 12 through the apertures 24, an obstruction means for solids and liquids is provided in the form of a closed loop configured structure. Preferably, as shown in Fig, 21, the obstruction means takes the form of a ring 65 of resilient open cell foam material, preferably a biodegradable material, but also usefully of any other resilient or also semi-rigid or rigid material allowing flatus gasses to pass therethrough, but hindering or preventing stomal effluent solids and liquids from passing therethrough.

The ring 65 operates as a pre-filter and is arranged between the aperture 5 of the face plate 1 and the coupling rings 6, 6a to allow flatus gasses to pass through the obstruction means into the chamber 22 and through the filter 17 and out of the aperture 18, but to hinder solids and liquids from passing into the chamber 22 so that the filter 17 is not clogged by said solids and liquids.

The ring 65 is attached to the inner bag wall 63a around the aperture 64 by means of an annular seam 66 of adhesive or weld such that the ring 65 is removed together with the inner bag 63 and flushed down a toilet.

Ideally, the material of the ring 65 is compressible and the axial extent of the ring 65 is slightly larger than the distance between the inner bag wall 63a and the carrier sheet 4 when the coupling rings 6 and 6a are engaged such that in said engaged position of the coupling rings the ring 65 is slightly axially compressed such that a pressure is exerted by the ring 65 on the strip 9 and carrier sheet 4 to ensure that no solids and liquids may pass between the ring 65 and the face plate 1.

In use, the release sheet 3 is removed from the adhesive layer 2, and the proximal surface of the layer 2 is applied to the peristomal skin of a wearer such that the stoma is received in the aperture 5. The collecting ostomy bags 61 and 63 are then attached to the faceplate 1 by engaging the coupling ring 6a of the bags with the matching coupling ring 6.

Stomal material consisting of solid material, liquid and flatus gas is then discharged from the stoma into the inner bag 63. The solid material and liquid falls down into the inner bag 63 while the flatus gas is led out through the ring 65 and the flatus gas passages defined by the inlet apertures 24, the compartment portion 22, the aperture 24 and the compartment portion 23, the filter 17 and the flatus gas outlet 18. The flow of the flatus gas is indicated by means of arrows.

The filter 17 is of the type disclosed in US Patent No. 6,506,184.
However, any other suitable type of deodorising filter may be used.

The coupling ring 6 is flexibly attached to the flexible plate 1 with the advantages inherent in this design as explained and disclosed in co-owned US patents No. 4,419,100 and 5,730,735.

When the inner bag 63 is full and is to be replaced by an empty bag, the coupling rings 6 and 6a are disengaged and a new set of inner and outer bags 61 and 63 with a new, clean pre-filter ring 65 is attached to the face plate 1. In this operation the ring 65 performs the further function of guiding the coupling ring 6a into engagement with the coupling ring 6, as the ring 65 protrudes proximally beyond the coupling ring 6a.

Hereby a new pre-filter ring 65, unsoiled by stomal solids and liquids is provided such that the flatus gas again can flow unhindered by solids and liquids adhered to the inner surface of the ring 65 to the filter 17.

Furthermore, the ring 65 prevents stomal solids and liquids from soiling the coupling ring 6a allowing the outer bag 63 to be folded together and disposed of in a pocket or bag without emitting any odours.

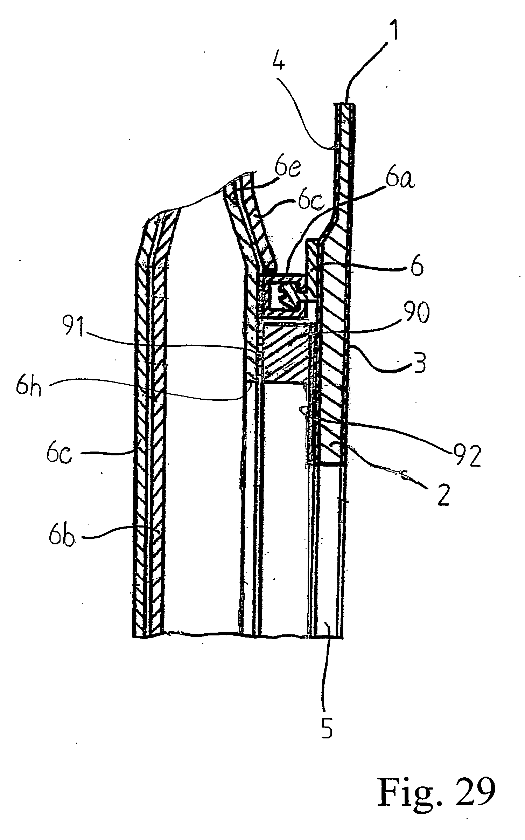

The ring 65 also prevents stomal solids and liquid from contacting the coupling ring 6 of the face plate 1 as well as a major region of the distal surface of the face plate 1 located between the coupling ring 6 and the stoma receiving aperture 5. This renders the cleaning of the face plate when replacing the pouches 61 and 63 much easier and more effective as only the region between the inner surface of the ring 55 and the stoma received in the aperture 5 is to be cleaned. Reference is made to Figs. 29-31 discussed below.

The material of the ring 65 may be any suitable porous, preferably at least slightly resilient, material such as open cell foam, non-woven material, textile, gauze and so on. The material is preferably hydrophobic and biodegradable or soluble under certain circumstances.

The currently preferred material for the ring 65 are fibres of 100% rayon coated with a paraffin wax (a paraffin emulsion Aurapel 374 supplied by the company Bayer Chemie). The ring has a thickness of approx. 9 mm and a weight of 35 oz/sqy and is supplied by the company High Tech Conversion.

The surface of the ring 65 facing the stoma receiving aperture 5 may be treated with a skin friendly product such as aloe vera or some other porous skin friendly cream such that any contact between the ring 65 and the stoma will bring the skin friendly material into contact with the stoma.

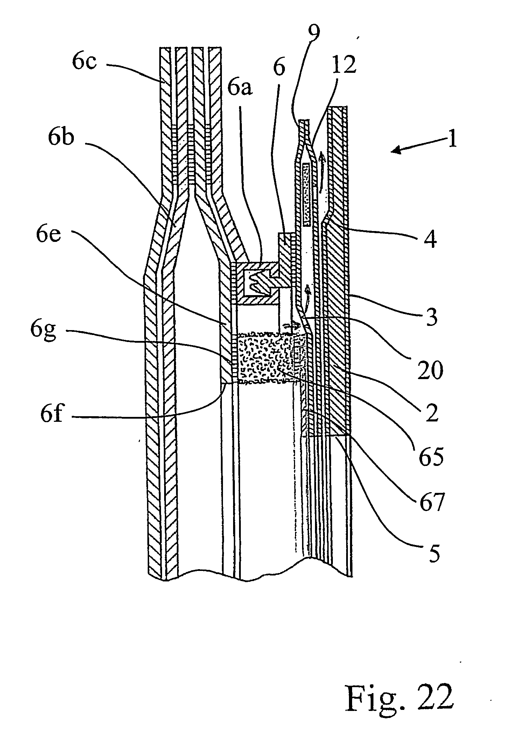

As shown most clearly in Fig. 22, the pre-filter ring 65 is attached to the proximal wall 6e of the bag 6b around the stoma receiving aperture 6f thereof by means of an adhesive seam or a heat seal 6g such that when replacing the bag with a new empty bag the ring 65 is also replaced as described above in connection with Fig. 21.

An annular plastic film 67 may be attached to the proximal surface of the ring 65 and be pressed against the face plate 1 by the ring 65. The film ring 67 prevents stomal material from soiling the region of the face plate 1 between the ring 65 and the stoma receiving aperture 5 such that when the pouch 6b is removed, the face plate 1 and coupling rings 6 and 6a are substantially clean.
The film ring 67 may also be applied to the pre-filter 65 in Fig. 21.

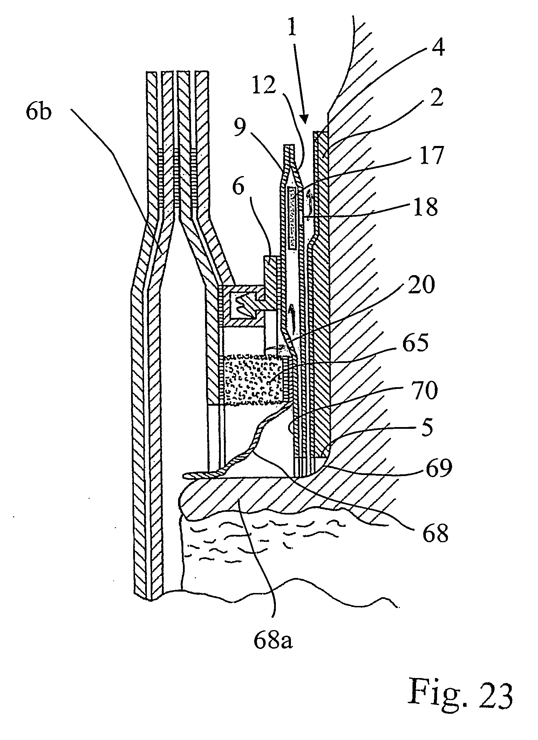

Referring now to Fig. 23, the embodiment shown therein is identical to the embodiment of Fig. 22 except that the annular plastic film in Fig. 23 lying flat against the carrier sheet 4 of the face plate 1, a sheath or cuff 68 of a plastic material is fastened to the pre-filter ring 65.

The sheath 68 abuts the circumferential surface of a stoma 68a received in the aperture 5 of the face plate 1 and thereby protects the stoma 68a and the peristomal skin surface 69 from contact with stomal material and also ensures that the distal surface region 70 of the face plate between the ring 65 and the stoma 69 is not soiled by stomal material such that when the pouch 6b with ring 65 and sheath 68 is removed for being replaced by a new pouch 6b with a new ring 65 with sheath 68, the necessity for cleaning the face plate is eliminated or dramatically reduced.

The material of the sheath may be any skin friendly material which preferably is plastically and/or elastically deformable.

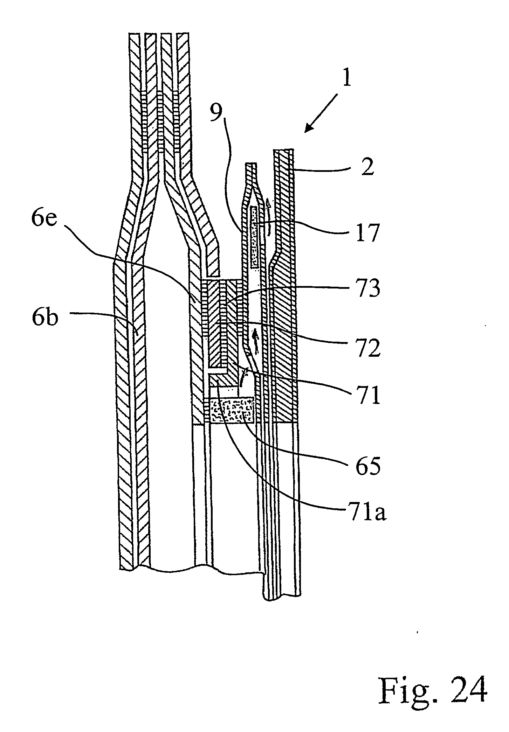

Referring now to Fig. 24, in this embodiment the coupling means for releasably attaching the bag 6b to the face plate 1 comprises a flanged ring 71 of relatively rigid material and a planar ring 72 also of relatively rigid material releasably attached to one another by means of a layer of releasably adhesive material 73, the rings 71 and 72 being fixedly attached by heat sealing to the filter pouch film 9 and to the proximal bag wall 6e, respectively. The flange 71a of the ring 71 serves to guide the ring 72 when mounting a new bag 6b on the face plate 1.

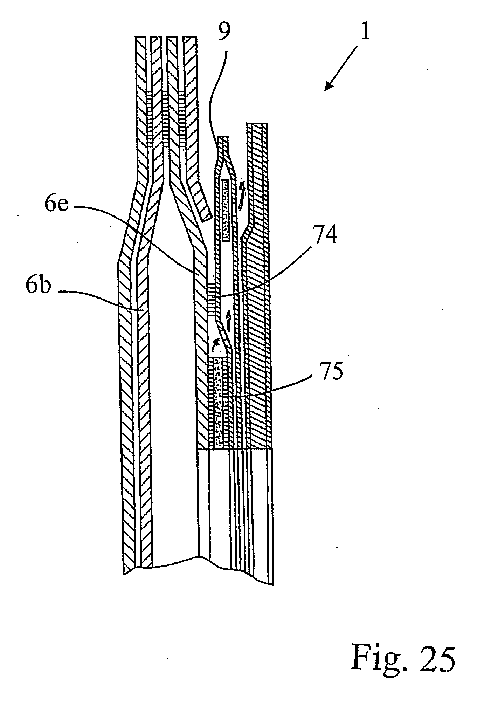

Referring now to Fig. 25, a so-called one-piece ostomy appliance is shown
where the bag 6b is fixedly attached to the face plate 1 by means of a heat seal or adhesive seam 74 between the filter pouch wall 9 and the proximal bag wall 6e.

A flat pre-filter ring 75 corresponding to ring 65 in Figs. 21-25 is fixedly attached to the proximal bag wall 6e and to filter pouch wall 9 such that all flatus gas is forced to flow through ring 75 as indicated by the arrows.

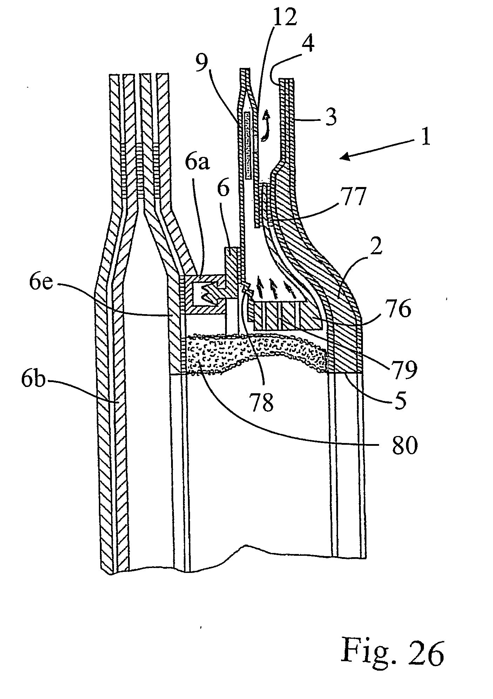

Referring now to Fig. 26, a so-called convex face plate 1 for use by persons with depressed or recessed stomas is shown wherein the adhesive layer 2 is constrained to the proximally convex form shown by means of a relatively rigid convex ring 76 fixedly attached to the carrier sheet 3 by means of a heat seal seam 77.

The convex ring 76 is flexibly attached to the coupling ring 6 by means of the filter pouch film 9 that is provided with annular accordion-like folds 78 for allowing the coupling ring 6 to move relative to the convex ring 76 which facilitates coupling and uncoupling of the rings 6 and 6a for replacement of the bag 6b.

Flatus gas passages 79 are provided through the convex ring 76 for allowing flatus gas to pass into the filter pouch 9, 12 as indicated by the arrows. The passages 79 are provided evenly distributed along at least the upper half of the convex ring.

A pre-filter ring 80 corresponding to pre-filter ring 65 in Figs. 21-23 is fixedly attached to the proximal pouch wall 6e and has a proximally tapering cross-section so as to reflect the reduced distance between the convex ring 76 and the stoma receiving aperture 5 such that a stoma received in the aperture does not come into too forceful contact with the ring 80.

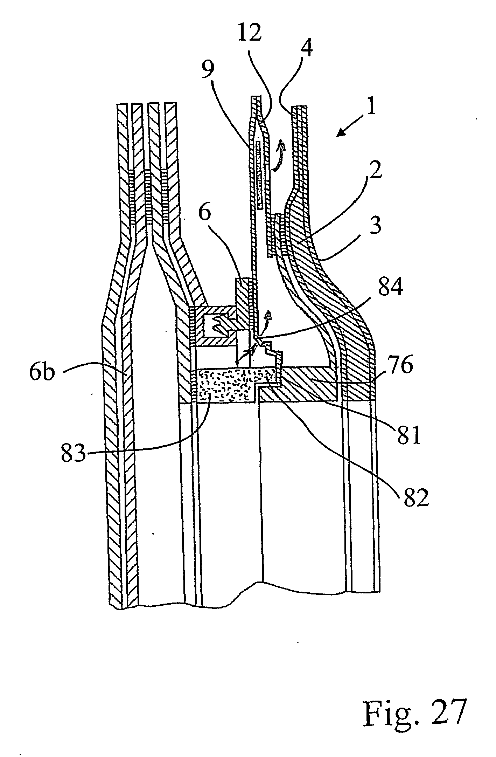

Referring now to Fig. 27, another embodiment of a convex ostomy appliance is shown wherein the convex ring 76 is provided with an annular recess 81 for receiving an annular flange 82 of a pre-filter ring 83 corresponding to pre-filter ring 80 in Fig. 26.

In this embodiment, the flatus gas flows into the filter chamber 9, 12 through one or more apertures 84 in the film 9 in the region between the coupling ring 6 and the convex ring 76 as indicated by the arrows.

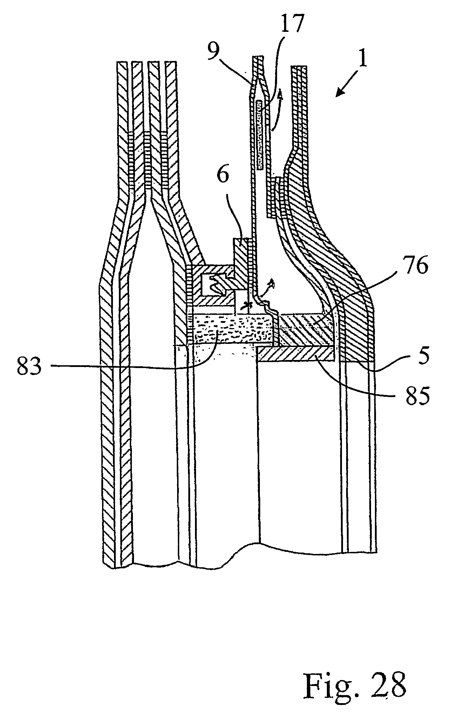

Referring now to Fig. 28, this embodiment is identical to Fig. 27 except that the pre-filter ring 83 does not have a flange and instead is kept in place on the convex ring 76 by means of an additional ring 85 fitting tightly inside the convex ring 76.

Referring now to Fig. 29, an ostomy appliance comprising a face plate 1 with an adhesive flexible plate 2, a release sheet 3, a carrier sheet 4 and a stoma receiving aperture 5, and an ostomy bag 6b attached to the face plate 1 by means of interlocking coupling rings 6 and 6a, is provided with means in the form of a separate, closed loop, preferably annular, axially elongated body for hindering or preventing contact between stomal discharge and the face plate distal surface and the coupling rings 6 and 6a.

In the Fig. 29 embodiment these means comprise an axially elongated body in the form of a ring 90 of a shape recoverable, resilient, compressible open cell or closed cell foam material or other material having similar properties fixedly attached to the ostomy bag proximal wall 6e around an opening 6h in said proximal wall 6e by means of an adhesive or heat sealing seam 91. The ring 90 is preferably hydrophobic.

An annular film 92 of plastic material is attached to the proximal surface of the ring 90 and extends to the edge of the aperture 5 such that the ring 90 and the film 92 protect substantially the entire area of the face plate between the coupling ring 6 and the aperture 5 from coming into contact with stomal discharge.

When the bag 6b is full of stomal discharge and is to be replaced, the coupling rings 6 and 6a are disengaged and the bag 6a with ring 90 and annular film 92 is removed. The necessity of cleaning the face plate 1 and coupling rings 6 and 6a after removal of the bag 6b and before attaching a new empty bag 6b is dramatically reduced or even totally eliminated.

The height of the ring 90 in the direction at right angles to the plane of the face plate 1, i.e. the axial dimension of the ring 90, should be slightly larger in an uncompressed state than the combined height of the coupling rings 6 and 6a so that the ring 90 is pressed against the face plate distal surface so that no stomal discharge can penetrate between the ring and the face plate.

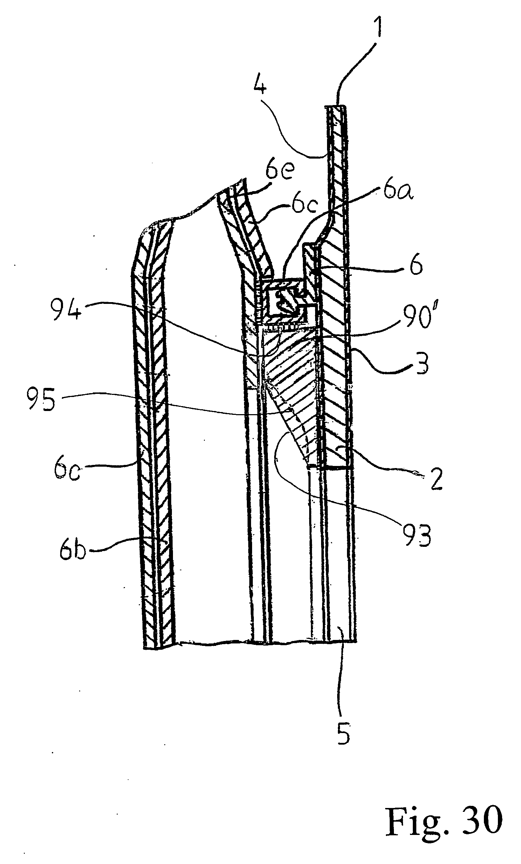

The width of the ring parallel to the face plate plane may be so large that the ring itself covers the face plate surface between the coupling rings and the aperture 5. Even though the width of the ring parallel to the face plate plane is less than the distance between the coupling rings 6, 6a and the edge of the aperture 5 it is not necessary that the annular film 92 be provided, although in such case some cleaning of the face plate is normally necessary.
Referring now to Fig. 30, this embodiment is identical to the Fig. 29 embodiment except that the ring 90' is so wide that it covers the entire area of the distal surface of the face plate between the coupling rings and the aperture 5, that the inwards facing surface 92 of the ring 90' is oblique, and the ring 90' is attached to the coupling ring 6a by means of an adhesive or heat seal seam 94.

The inwardly facing surface of the ring 90' may be inwardly concave as indicated by the dotted line 95 such that the height or axial dimension of the ring 90' in the region adjacent the aperture 5 is even smaller than in the embodiment with the oblique surface 93.

It is often that case that the individual ostomate adapts the contour of the face plate aperture 5 to the size and/or shape of his or her stoma by cutting the face plate with a scissors or a cutting tool. By reducing the height of the ring 90' near the aperture 5 it is rendered easier for the individual ostomate to also adapt the inner edge of the ring 90' correspondingly such that the ring 90' does not press too much against the stoma when mounted on the face plate. In the Fig. 29 embodiment it is relatively easy to adapt the inner edge of the relatively thin annular film 92.

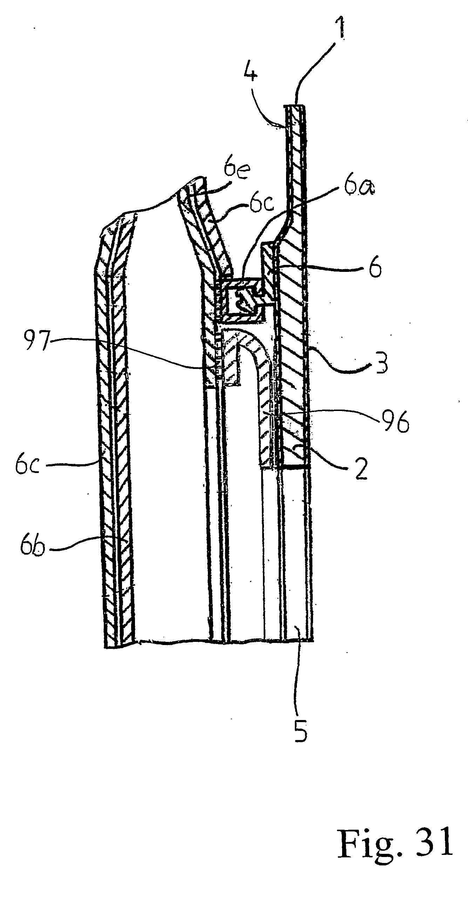

Referring now to Fig. 31, this embodiment is identical to the Fig. 29 embodiment except that the ring 90 with annular film 92 has been substituted by a convoluted ring 96 of a flexible plastic material attached to the proximal bag wall 6e by means of an adhesive or a heat sealing seam 97. The thickness of the ring may vary such that the region near the aperture 5 is thinnest thereby easing the adaptation of the inner edge of the ring 96 to the individual stoma as explained above.

The ostomy bags in the Figs. 29-31 embodiments are single bag appliances with the bag attached to the face plate by means of interlocking coupling rings,

However, the rings 90 and 90' as well as 96 may just as well be applied to bag in bag appliances such as shown in Fig. 21 and appliances wherein the bag or bags are releasably adhered to the face plate by means of adhesive such as in Fig. 24.

Many variations and modifications are conceivable within the scope of the appended claims.

Thus, the pre-filter ring 65 and 80 may for instance be substituted by a perforated or gas permeable ring of relatively rigid plastic, flexible plastic film or cardboard or other suitable, preferably biodegradable material and preferably provided at the proximal edge region thereof with a flexible annular apron resiliently contacting the distal surface of the face plate between the coupling ring 6 and the stoma aperture 5.

The ring 65 may be configured such that the inner surface facing the stoma is inclined such that the proximal surface of the ring contacting the face plate is radially wider than the distal surface, preferably such that the inner rim of said proximal surface is adjacent the rim of the stoma receiving aperture of the face plate such that the ring 65 itself may perform the "wiping" or "sweeping function described above when the bag or bags are removed.

Instead of attaching the pre-filter ring 65, 80, 83 to the proximal wall 6e of the bag, the ring may be attached to the pouch coupling ring 6a.

The pre-filter rings need not necessarily be attached to the bag wall or the coupling ring, but may be a separate ring that is placed inside the coupling ring 6 prior to attaching the coupling ring 6a to the coupling ring 6.

The location of the venting apertures 20 may be chosen in many manners, but it is preferred that the venting apertures be provided adjacent the top half of the coupling rings such that if the user of the face plate takes a shower without having a bag mounted on the face plate, water from the shower will have more difficulty in penetrating through the venting apertures into the filter pouch and block the filter which preferably also should be placed adjacent the top of the coupling rings.

The annular film 92 of the Fig. 29 embodiment may be substituted by the sheath 68 of the Fig. 23 embodiment.

It will be obvious to persons skilled in the art that features from one embodiment may be combined with features of another embodiment as long as resulting combination embodiments are within the scope of the appended claims.

For instance, the layer of non-woven material described in connection with Fig. 4A may applied to all the embodiments in Figs. 1-2 and 4-28.

## Claims

1. An adhesive face plate (1) for securing an ostomy bag (6b) to the skin of an ostomy patient, said face plate (1) comprising:
- a flexible plate (2) with a bodyside or proximal surface and a distal surface and comprising a layer of a skin friendly adhesive material and a stoma receiving aperture (5) for receiving a stoma of said patient,
- generally annular face plate coupling means (6) having an inner (8) and an outer (7) periphery and adapted for fixedly or releasably engaging corresponding bag coupling means (6) of said ostomy bag (6b); said face plate coupling means (6) being secured to said flexible plate (2) with the plane of said face plate coupling means (6) generally parallel to the plane of said flexible plate and generally concentric with said aperture for receiving said stoma, and
- a filter (17) for de-odorising flatus gas,
**characterized by**
- one or more flatus gas passages extending between the face plate coupling means (6) and said flexible plate (2) from said inner periphery (8) to said outer periphery (7) and through said filter (17).

2. A face plate according to claim 1, wherein said face plate coupling means (6) and staid bag coupling means (6a) are mutually matching coupling rings adapted for releasably engaging one another.

3. A face plate according to claim 1, wherein said face plate coupling means (6) and said bag coupling means (6a) are mutually matching surfaces adapted for being releasably or fixedly adhered to one another by means of an adhesive (73) or fixedly adhered to one another by means a heat seal seam (74).

4. A face plate according to claim 3, wherein one or both of said mutually matching surfaces are provided by a ring (71, 72) of a relatively rigid material.

5. A face plate According to any of the preceding claims and further comprising a compartment (16) having an exterior portion (16b) located, outside said outer periphery (7) and an interior portion (16a) located between said face plate coupling means (6) and said flexible plate (2), a flatus gas outlet (18) being provided in a wall (12) of said exterior portion, said compartment forming part of said flatus gas passage, said fitter (17) being located within staid compartment such that said flatus gas outlet is obstructed by said filter (17).

6. A face plate according to claim 5, wherein said exterior portion (16b) of said compartment (16) is defined by a first film strip (9) of plastic material, preferably adapted to define a pouch.

7. A face plate according to claims 6, wherein said first film strip (9) has a first edge attached to said face plate coupling means (6) and a second edge opposed said first edge attached to said flexible plate (2) so as to allow said flatus gas passage to extend between said first and second edges.

8. A face plate according to claim 7, wherein said first film strip (9) is composed of a second and a third film strip of said plastic material, said second and third film strips comprising said first edge and said second edge, respectively, and said second and third film strips being attached to one another along the edges thereof opposed to said first and second edges, respectively.

9. A face plate according to claims 6, wherein said first film strip (9) has a first edge attached to staid coupling ring and a second edge opposed said first edge-attached to said flexible plate outside said outer periphery.

10. A face plate according to any of the claims 5-9, wherein said exterior portion (16b) of said compartment (16) extends along part of the circumference of said outer periphery (7).

11. A face plate according to any of the claims 5-9, wherein said exterior portion (16b) of said compartment (16) extends along the entire circumference of said outer periphery (7).

12. A face plate according to any of the claims 3-11, wherein a first edge of a fourth film strip (12) of plastic material is attached to said face plate coupling means (6) and a second edge of said fourth film strip (12) opposed to said first edge is attached to said flexible plate (2) inside said outer periphery, at least one flatus gas inlet aperture (19, 20) provided in said fourth film strip (12) constituting a flatus gas inlet to a corresponding one of said at least one flatus gas passages.

13. A face plate according to claim 12, wherein said fourth film strip (12) is integral with said first film strip (9).

14. A face plate according to claim 12 or 13, wherein said flatus gas inlet aperture (19, 19a, 19b, 20, 24) is located remote from said gas outlet aperture (18).

15. A face plate according to claim 14, wherein open ceil foam material (25) is arranged in said compartment (16) between said flatus gas inlet aperture (19, 20) and said flatus gas outlet (18) such that substantially the entire cross sectional area of the compartment (16) is filled with said foam material at least one point between said flatus gas inlet aperture (19, 20) and said flatus-gas outlet (18).

16. A face plate according to any of the preceding claims, wherein obstructing means (65) for hindering or preventing stomal fluids and liquids entering said flatus gas passage is provided adjacent said inner periphery (8).

17. A face plate according to claim 16, wherein said obstructing means (65) comprises a gas permeable, liquid impermeable film arranged such that said film covers the inlet to said flatus gas passage.

18. A face plate according to claim 17 and any of the claims 12-16, wherein said film is attached to said fourth film strip (12) at a region surrounding said flatus gas inlet aperture (20).

19. A face plate according to claim 17 and any of the claims 12-16, wherein said fourth film strip (12) is constituted by said gas permeable, liquid impermeable film.

20. A face plate according to any of the claims 16-19, wherein said obstructing means (65) comprises a fifth film strip with one longitudinal edge attached to the flexible plate (2, 3, 4) inside said inner periphery (8) along the entire circumference of said coupling ring (6), the opposed longitudinal edge of said fifth film strip being free to move such that an annular collar extending away from said flexible plate is formed by said fifth film strip.

21. A face plate according to any of the claims 16-20. wherein the obstructing means (65) comprises a sixth film strip with one longitudinal edge attached to said coupling ring (6) along part of the total circumference thereof and extending inwards from said inner periphery (8), the opposed longitudinal edge of said sixth film strip being free to move such that a curtain extending generally parallel to the flexible plate is formed by said sixth film strip.

22. A face plate according to claim 21, wherein said inlet to said flatus gas passage is located at said part of the total circumference, preferably near the middle thereof.

23. A face plate according to claim 16, wherein said obstructing means (65) composes an elongated body of a gas permeable, preferably hydrophobic material such as open cell foam or non-woven material, said body having an arcuate, preferably closed loop and preferably annular shape and being located between said face plate coupling means (6) and said stoma receiving aperture (5).

24. A face plate according to claim 23, wherein said body abuts said distal surface of said flexible plate (2, 3, 4) along substantially the entire length of said body.

25. A face plate according to claim 23 or 24, wherein an annular film (67) is attached to the proximal surface of said body and covers at least part, preferably substantially all the region of said distal surface located between said body and said stoma receiving aperture (5).

26. A face plate according to claim 25, wherein said annular film (67) extends away from said body beyond the periphery of said stoma receiving aperture (5) such that a stoma (69) received in said stoma receiving aperture (5) will be partly covered by said annular film in the form of a sheath or cuff (68) for said stoma.

27. A face plate according to any of the claims 5-26, wherein a labyrinth is provided in said compartment (to) between the inlet (19, 20) to said flatus gas passage and said filter (17) such that said flatus gas passage comprises said labyrinth.

28. A face plate according to claim 27 and any of the claims 12-26, wherein said labyrinth comprises a seventh film strip (21) of plastic material having a first edge attached to said flexible plate (2, 3; 4) together with said second edge of said fourth film strip (12), said seventh film strip (21) having a second edge strip opposed to said first edge thereof attached to said first film strip (9) such that the compartment defined between said first film strip (9) and said fourth film strip (12) is subdivided into two chambers (22, 23), at least one flatus gas flow aperture (24) being provided in said seventh film strip.

29. A face plate according to any of the claims 12-26, wherein spacing means (28, 29) are provided between said fourth film strip (12) and said first film strip (9) at least adjacent said flatus gas inlet aperture (19, 20) such that a space is provided between said first (9) and fourth (12) film strips to allow flatus gas to flow through said flatus gas inlet aperture (91, 20) unimpeded by any adhesion between said first (9) and fourth (12) film strips.

30. A face plate according to any of the claims 28, wherein spacing means (28, 29) arse provided between said fourth film strip (12) and said seventh film strip (21) adjacent said flatus gas inlet aperture (19, 20) such that a space is provided between said first (9) and seventh (21) film strips to allow flatus gas to flow through said flatus gas inlet aperture (19, 20) unimpeded by any adhesion between said seventh (21) and fourth (12) film strips.

31. A face plate according to claim 29 or 30, wherein said spacing means (28, 29) comprises a strip of non-woven material.

32. A face plate according to any of the claims 1-4, wherein said filter (42) is annular and is arranged between said face plate coupling means (6) and said flexible plate (2, 3, 4).

33. A face plate according to claim 32, wherein said coupling ring (6) is attached to said flexible plate (4) by means of said annular filter (42).

34. An adhesive face plate (1) for securing an ostomy bag (6b) to the skin of an ostomy patient, said faceplate comprising:
- a flexible plate (2) with a bodyside or proximal surface and a distal surface and comprising a layer of a skin friendly adhesive material and a stoma receiving aperture (5) for receiving a stoma of said patient,
- generally annular face plate coupling means (6) having an inner (8) and an outer (7) periphery and adapted for fixedly or releasably engaging corresponding bag coupling means (6) of said ostomy bag (6b), said face plate coupling means (6) being secured to said flexible plate (2) with the plane of said face plate coupling means (6) generally parallel to the plane of said flexible plate (2) and generally concentric with said aperture (5) for receiving said stoma, and
- a filter (17) for de-odorising flatus gas,
**characterised by**
- a flatus gas venting compartment (16) in the form of a pouch or bag made of a first film (12) of plastic Material and extending outward beyond said outer periphery (7), said first film (12) being provided with a flatus gas outlet aperture (18), and
- a flatus gas passage extending from said inner periphery (8) into the interior of said venting pouch (16) and through both said filter (17) and said flatus gas outlet aperture (18), said filter being arranged such that substantially all said flatus gas vented through said flatus gas aperture (18) flows through said filter (17).

35. A face plate according to claim 34, wherein said face plate coupling means (6) and said bag coupling means (6a) are mutually matching coupling rings adapted for releasably engaging one another.

36. A face plate according to claim 34, wherein said face plate coupling means (6) and said bag coupling means (6a) are mutually matching surfaces adapted for being releasably or fixedly adhered to one another by means of an adhesive (73) or fixedly adhered to one another by means a heat seal seam (74).

37. A face plate according any of the claims 33-36, wherein said flatus gas passage extends between said face plate courting means (6) and said flexible plate (2, 3, 4).

38. A face plate according to any of the claims 33-36, wherein said flatus gas passage extends at least partly through a conduit (50) extending through a portion of said face plate coupling means (6) from a conduit inlet to a conduit outlet,

39. A face plate according to claim 38, wherein one edge of said venting pouch (16) is attached to a body side or proximal surface (53) of said face plate coupling means (6) facing said flexible plate and the opposed edge of said venting pouch is attached to a distal surface (54) of said face plate coupling means (6) facing away from said flexible plate, said conduit outlet being located between said proximal and distal surfaces such that flatus gas exiting from said conduit enters said venting pouch (16).

40. A face plate according to any of the claims 36-39, wherein said pouch (16) extends along part of the circumference of said outer periphery (7).

41. A face plate according to any of the claims 36-39, wherein said pouch (16) extends along the entire circumference of said outer periphery (7).

42. A face plate according to any of the claims 34-41, wherein a first edge of a first film strip (9) of plastic material is attached to said coupling ring (6) and a second edge of said first film strip (9) opposed to said first edge is attached to said flexible plate (2,3. 4) inside said outer periphery (7), at least one flatus gas inlet aperture (19. 20) being provided in said first film strip.

43. A face plate according to any of the claims 34-42, wherein open cell foam material, (25) is arranged in said pouch upstream of said filter (17) such that substantially the entire cross sectional area of said vending pouch (16) is filled with said foam material (25) at at least one point.

44. A face plate according to any of the claims 34-43, wherein obstructing means (65) for hindering or preventing stomal fluids and liquids entering said flatus gas passage is provided adjacent said inner periphery (8).

45. A face plate according to claim 44, wherein said obstructing means (65) comprises a gas permeable, liquid impermeable film arranged such that said film covers the inlet to said flatus gas passage.

46. A face plate according to claim 44 and 45, wherein said film is attached to said first film strip (9) at a region surrounding said flatus gas inlet aperture (20).

47. A face plate according to claim 45 and claim 42, wherein said first film strip (9) is constituted by said gas permeable, liquid impermeable firm.

48. A face plate according to any of the claims 44-47, wherein said obstructing means (65) comprises a second film strip with one longitudinal edge attached to the flexible plate (2, 3, 4) inside said inner periphery (8) along the entire circumference of said coupling ring (6), the opposed longitudinal edge of said second film strip being free to move such that an annular collar extending away from said flexible plate (2, 3, 4) is formed by said second film strip.

49. A face plate according to any of the claims 44-47, wherein the obstructing means (65) comprises a third film strip with one longitudinal edge attached to said coupling ring (6) along part of the total circumference thereof and extending inwards from said inner periphery (8), the opposed longitudinal edge of said third film strip being free to move such that a curtain extending generally parallel to the flexible plate is formed by said third film strip.

50. A face plate according to claim 49, wherein said inlet (20) to said flatus gas passage is located at said part of the total circumference, preferably near the middle thereof.

51. A face plate according to claim 44, wherein said obstructing means (65) comprises an elongate body of a gas permeable, preferably hydrophobic material such as open cell foam or non-woven material, said body having an arcuate, preferably closed loop and preferably annular shape and being located between said face plate coupling means (6) and said stoma receiving aperture (5).

52. A face plate according to claim 51, wherein said body abuts said distal surface of said flexible plate (2, 3, 4) along substantially the entire length of said body.

53. A face plate according to claim 51 or 52, wherein an annular film (67) is attached to the proximal surface of said body and covers at least part, preferably substantially all the region of said distal surface located between said body and said stoma receiving aperture (5).

54. A face plate according to claim 53, wherein said annular film (67) extends away from said body beyond the periphery of said stoma receiving aperture (5) such that a stoma (69) received in said stoma receiving aperture (5) will be partly covered by said annular film (67) in the form of a sheath or cuff (68) for said stoma.

55. A face plate according to any of the claims 34-53, wherein a labyrinth is provided in said pouch (16) between the inlet (20) to said flatus gas passage and said filter (17) such that said flatus gas passage comprises said labyrinth.

56. A face plate according to claim 55 and any of the claims 40-54, wherein said labyrinth comprises a fourth film strip (21) of plastic material arranged and adapted to subdivide said pouch (16) into two chambers (22 and 23), one chamber (22) communicating directly with said flatus gas passage inlet and the other chamber (23) containing said filter, at least one flatus gas flow aperture (24) being provided in said fourth film strip.

57. A face plate according to claim 38, wherein spacing means (28, 29) are provided at least adjacent said conduit outlet (50) such that a space is provided between the film walls of said pouch to allow flatus gas to flow through said flatus gas conduit (50) unimpeded by any adhesion between said film walls.

58. A face plate according to claim 42, wherein spacing means (28, 29) are provided at least adjacent said flatus gas inlet aperture (19, 20) such that a space is provided downstream from said flatus gas inlet aperture (19, 20) to allow flatus gas to flow through said flatus gas inlet aperture (19, 20) unimpeded by any adhesion between said first film strip and a pouch wall or the flexible plate.

59. A face plate according to claim 57 or 58, wherein said spacing means (28, 29) comprises a strip of non-woven material.

60. A face plate according to any of the preceding claims, wherein said proximal surface of said flexible plate (2, 3, 4) is convex, a relatively rigid convex ring (76) being attached to said distal surface of said flexible plate.

61. A face plate according to claim 60, wherein flatus gas passages are provided through said convex ring (76).

62. An ostomy appliance for receiving human stomal discharge comprising in combination an adhesive face plate according to any of the preceding claims and an ostomy bag for receiving human stomal discharge releasably or fixedly attached to said face plate.

63. An ostomy appliance according to claim 62, wherein said ostomy bag (6b) comprises a single bag of a gas and liquid impermeable material.

64. An ostomy appliance according to claim 62, wherein said ostomy bag comprises two bags, an outer bag (61) of a gas and liquid impermeable material and an inner bag (63) of a flexible material and contained within said outer bag (61).

65. An ostomy appliance according to claim 63 as dependent on any of the claims 23-26 or 51- 35 54, wherein said elongate body (90) is attached to said ostomy bag (6b) and or said bag coupling means (6a) such that removal of said ostomy bag (6b) from said face plate (1) entails removal of said elongate body from said face plate.

66. An ostomy appliance according to claim 64 as dependent on any of the claims 23-26 or 51-54, wherein said elongate body (90) is attached to said inner ostomy bag (63) such that removal of said inner ostomy bag (63) from said face plate (1) entails removal of said elongate body (90) from said face plate (1).

## Patentansprüche

1. Selbstklebende Stirnplatte (1) zum Befestigen eines Ostomiebeutels (6b) an der Haut eines Ostomiepatienten, wobei die Stirnplatte (1) enthält:
- eine flexible Platte (2) mit einer Körperseite oder einer proximalen Oberfläche und einer distalen Oberfläche und enthaltend eine Schicht eines hautfreundlichen Haftmaterials und eine Stoma-Aufnahmeöffnung (5) für die Aufnahme eines Stomas des Patienten,
- eine im wesentlichen ringförmige Stirnplatten-Kopplungseinrichtung (6), die einen inneren (8) und einen äußeren (7) Umfang hat und dazu eingerichtet ist, fest oder lösbar mit einer entsprechenden Beutelkopplungseinrichtung (6) des Ostomiebeutels (6b) in Eingriff zu gelangen, wobei die Stirnplatten-Kopplungseinrichtung (6) an der flexiblen Platte (2) derart befestigt ist, dass die Ebene der Stirnplatten-Kopplungseinrichtung (6) im wesentlichen parallel zur
Ebene der flexiblen Platte verläuft und im wesentlichen konzentrisch mit der Öffnung zur Aufnahme des Stomas ist, und
- ein Filter (17) zum Deodorieren eines Flatusgases,
**gekennzeichnet durch**
- wenigstens einen Flatusgasleitungsweg, der sich zwischen der Stirnplatten-Kopplungseinrichtung (6) und der flexiblen Platte (2) von dem Innenumfang (8) zu dem Außenumfang (7) und **durch** das Filter (17) erstreckt.

2. Stirnplatte nach Anspruch 1, bei der die Stirnplatten-Kopplungseinrichtung (6) und die Beutelkopplungseinrichtung (6a) wechselseitig zueinander passende Kopplungsringe sind, die dazu eingerichtet sind, miteinander lösbar in Eingriff zu gelangen.

3. Stirnplatte nach Anspruch 1, bei der die Stirnplatten-Kopplungseinrichtung (6) und die Beutelkopplungseinrichtung (6a) wechselseitig zueinander passende Oberflächen sind, die dazu eingerichtet sind, mit Hilfe eines Haftmittels (73) lösbar oder fest aneinander zu haften oder mit Hilfe einer Schweißnaht (74) fest aneinander zu haften.

4. Stirnplatte nach Anspruch 3, bei der wenigstens eine der wechselseitig zueinander passenden Oberflächen durch einen Ring (71, 72) eines relativ steifen Materials bereitgestellt ist.

5. Stirnplatte nach einem der vorhergehenden Ansprüche und weiterhin enthaltend ein Abteil (16), das einen äußeren Abschnitt (16b), der sich außerhalb des Außenumfangs (7) befindet, und einen inneren Abschnitt (16a) hat, der sich zwischen der Stirnplatten-Kopplungseinrichtung (6) und der flexiblen Platte (2) befindet, und einen Flatusgasauslass (18), der in einer Wand (12) des äußeren Abschnittes vorgesehen ist, wobei das Abteil einen Teil des Flatusgasleitungsweges bildet und das Filter (17) innerhalb des Abteils derart angeordnet ist, dass der Flatusgasauslass durch das Filter (17) versperrt ist.

6. Stirnplatte nach Anspruch 5, bei der der äußere Abschnitt (16b) des Abteils (16) durch einen ersten Folienstreifen (9) eines Kunststoffmaterials definiert ist, der vorzugsweise dazu eingerichtet ist, eine Tasche zu bilden.

7. Stirnplatte nach Anspruch 6, bei der der erste Folienstreifen (9) einen ersten Rand, der an der Stirnplatten-Kopplungseinrichtung (6) befestigt ist, und einen zweiten Rand, gegenüberliegend dem ersten Rand, hat, der an der flexiblen Platte (2) befestigt ist, so dass sich der Flatusgasleitungsweg zwischen dem ersten und dem zweiten Rand erstrecken kann.

8. Stirnplatte nach Anspruch 7, bei der der erste Folienstreifen (9) aus einem zweiten und einem dritten Folienstreifen des Kunststoffmaterials besteht, wobei der zweite und der dritte Folienstreifen den ersten bzw. den zweiten Rand enthalten und der zweite sowie der dritte Folienstreifen entlang ihrer Ränder, gegenüberliegend dem ersten bzw. dem zweiten Rand, miteinander verbunden sind.

9. Stirnplatte nach Anspruch 6, bei der der erste Folienstreifen (9) einen ersten Rand, der an dem Kopplungsring angebracht ist, und einen zweiten Rand, gegenüberliegend dem ersten Rand, hat, der an der flexiblen Platte außerhalb des Außenumfangs angebracht ist.

10. Stirnplatte nach einem der Ansprüche 5 bis 9, bei der sich der äußere Abschnitt (16b) des Abteils (16) entlang eines Teils der Ausdehnung des Außenumfangs (7) erstreckt.

11. Stirnplatte nach einem der Ansprüche 5 bis 9, bei der sich der äußere Abschnitt (16b) des Abteils entlang der gesamten Ausdehnung des Außenumfangs (7) erstreckt.

12. Stirnplatte nach einem der Ansprüche 3 bis 11, bei der ein erster Rand eines vierten Folienstreifens (12) eines Kunststoffmaterials an der Stirnplatten-Kopplungseinrichtung (6) angebracht ist und ein zweiter Rand des vierten Folienstreifens (12), gegenüberliegend dem ersten Rand, an der flexiblen Platte (2) innerhalb des Außenumfangs angebracht ist und wenigstens eine Flatusgaseinlassöffnung (19, 20), die in dem vierten Folienstreifen (12) vorgesehen ist, einen Flatusgaseinlass zu einem entsprechenden des wenigstens einen Flatusgasleitungsweges bildet.

13. Stirnplatte nach Anspruch 12, bei der der vierte Folienstreifen (12) integraler Bestandteil des ersten Folienstreifens (9) ist.

14. Stirnplatte nach Anspruch 12 oder 13, bei der die Flatusgaseinlassöffnung (19, 19a, 19b, 20, 24) entfernt von der Gasauslassöffnung (18) angeordnet ist.

15. Stirnplatte nach Anspruch 14, bei der ein offenes Zellschaummaterial (25) in dem Abteil (16) zwischen der Flatusgaseinlassöffnung (19, 20) und dem Flatusgasauslass (18) derart angeordnet ist, dass im wesentlichen die gesamte Querschnittsfläche des Abteils (16) mit dem Schaummaterial wenigstens an einem Punkt zwischen der Flatusgaseinlassöffnung (19, 20) und dem Flatusgasauslass (18) gefüllt ist.

16. Stirnplatte nach einem der vorhergehenden Ansprüche, bei der eine Absperreinrichtung (65) benachbart des Innenumfangs (8) vorgesehen ist, die einen Eintritt stomaler Fluids und Flüssigkeiten in den Flatusgasleitungsweg behindert oder vermeidet.

17. Stirnplatte nach Anspruch 16, bei der die Absperreinrichtung (65) eine gasdurchlässige, flüssigkeitsundurchlässige Folie enthält, die derart angeordnet ist, dass die Folie den Einlass in den Flatusgasleitungsweg bedeckt.

18. Stirnplatte nach Anspruch 17 und einem der Ansprüche 12 bis 16, bei der die Folie am vierten Folienstreifen (12) in einem Bereich angebracht ist, der die Flatusgaseinlassöffnung (20) umgibt.

19. Stirnplatte nach Anspruch 17 und einem der Ansprüche 12 bis 16, bei der der vierte Folienstreifen (12) aus der gasdurchlässigen, flüssigkeitsundurchlässigen Folie besteht.

20. Stirnplatte nach einem der Ansprüche 16 bis 19, bei der die Absperreinrichtung (65) einen fünften Folienstreifen enthält, dessen einer Längsrand an der flexiblen Platte (2, 3, 4) innerhalb des Innenumfangs (8) entlang des gesamten Umfangs des Kopplungsringes (6) angebracht ist, wobei sich der gegenüberliegende Längsrand des fünften Folienstreifens derart frei bewegen kann, dass ein ringförmiger Kranz, der sich von der flexiblen Platte weg erstreckt, durch den fünften Folienstreifen ausgebildet ist.

21. Stirnplatte nach einem der Ansprüche 16 bis 20, bei der die Absperreinrichtung (65) einen sechsten Folienstreifen enthält, dessen einer Längsrand an dem Kopplungsring (6) entlang eines Teils seines gesamten Umfangs angebracht ist und sich vom Innenumfang (8) nach innen erstreckt, wobei sich der gegenüberliegende Längsrand des sechsten Folienstreifens derart frei bewegen kann, dass eine Abdeckschürze, die sich im wesentlichen parallel zu der flexiblen Platte erstreckt, durch den sechsten Folienstreifen ausgebildet ist.

22. Stirnplatte nach Anspruch 21, bei der der Einlass in den Flatusgasleitungsweg an diesem Teil des gesamten Umfangs, vorzugsweise in der Nähe der Mitte desselben angeordnet ist

23. Stirnplatte nach Anspruch 16, bei der die Absperreinrichtung (65) einen länglichen Körper eines gasdurchlässigen, vorzugsweise wasserabweisenden Materials, wie etwa einen offenen Zellschaum oder ein Vliesmaterial, enthält, wobei dieser Körper eine bogenförmige, vorzugsweise geschlossene kreisförmige und vorzugsweise ringförmige Gestalt hat und sich zwischen der Stirnplatten-Kopplungseinrichtung (6) und der Stoma-Aufnahmeöffnung (5) befindet.

24. Stirnplatte nach Anspruch 23, bei der der Körper an die distale Oberfläche der flexiblen Platte (2, 3, 4) im wesentlichen entlang der gesamten Länge des Körpers anschlägt.

25. Stirnplatte nach Anspruch 23 oder 24, bei der eine ringförmige Folie (67) an der proximalen Oberfläche des Körpers angebracht ist und wenigstens einen Teil, vorzugsweise jedoch den gesamten Bereich der distalen Oberfläche bedeckt, die sich zwischen dem Körper und der Stoma-Aufnahmeöffnung (5) befindet.

26. Stirnplatte nach Anspruch 25, bei der sich die ringförmige Folie (67) weg von dem Körper über den Umfang der Stoma-Aufnahmeöffnung (5) hinaus derart erstreckt, dass ein Stoma (69), das in der Stoma-Aufnahmeöffnung (5) aufgenommen ist, teilweise von der ringförmigen Folie in Gestalt einer Stulpe oder Manschette (8) für das Stoma bedeckt ist.

27. Stirnplatte nach einem der Ansprüche 5 bis 26, bei der ein Labyrinth in dem Abteil (16) zwischen dem Einlass (19, 20) in den Flatusgasleitungsweg und dem Filter (17) derart vorgesehen ist, dass der Flatusgasleitungsweg das Labyrinth enthält.

28. Stirnplatte nach Anspruch 27 und einem der Ansprüche 12 bis 26, bei der das Labyrinth einen siebten Folienstreifen (21) eines Kunststoffmaterials enthält, bei dem ein erster Rand an der flexiblen Platte (2, 3, 4) zusammen mit dem zweiten Rand des vierten Folienstreifens (12) angebracht ist, wobei der siebte Folienstreifen (21) einen zweiten Randstreifen gegenüberliegend dessen ersten Rand hat, der an dem ersten Folienstreifen (9) derart angebracht ist, dass das Abteil, das zwischen dem ersten Folienstreifen (9) und dem vierten Folienstreifen (12) gebildet ist, in zwei Kammern (22, 23) unterteilt ist, wobei wenigstens eine Flatusgasströmungsöffnung (24) in dem siebten Folienstreifen vorgesehen ist.

29. Stirnplatte nach einem der Ansprüche 12 bis 26, bei der Abstandshalteeinrichtungen (28, 29) zwischen dem vierten Folienstreifen (12) und dem ersten Folienstreifen (9) wenigstens benachbart der Flatusgaseinlassöffnung (19, 20) derart vorgesehen sind, dass ein Zwischenraum zwischen dem ersten (9) und dem vierten (12) Folienstreifen geschaffen ist, damit Flatusgas durch die Flatusgaseinlassöffnung (19, 20) ungehindert durch eine Haftung zwischen dem ersten (9) und dem vierten (112) Folienstreifen strömen kann.

30. Stirnplatte nach Anspruch 28, bei der die Abstandshalteeinrichtungen (28, 29) zwischen dem vierten Folienstreifen (12) und dem siebten Folienstreifen (21) benachbart der Flatusgaseinlassöffnung (19, 20) derart vorgesehen sind, dass ein Zwischenraum zwischen dem ersten (9) und dem siebten (21) Folienstreifen gebildet ist, damit Flatusgas durch die Flatusgaseinlassöffnung (19, 20) ungehindert durch eine Haftung zwischen dem siebten (21) und dem vierten (12) Folienstreifen strömen kann.

31. Stirnplatte nach Anspruch 29 oder 30, bei der die Abstandshalteeinrichtungen (28, 29) einen Streifen eines Vliesmaterials enthalten.

32. Stirnplatte nach einem der Ansprüche 1 bis 4, bei der das Filter (42) ringförmig ist und zwischen der Stirnplatten-Kopplungseinrichtung (6) und der flexiblen Platte (2, 3, 4) angeordnet ist.

33. Stirnplatte nach Anspruch 32, bei der der Kopplungsring (6) an der flexiblen Platte (4) mit Hilfe des ringförmigen Filters (42) angebracht ist.

34. Selbstklebende Stirnplatte (1) zum Befestigen eines Ostomiebeutels (6b) an der Haut eines Ostomiepatienten, wobei die Stirnplatte enthält:
- eine flexible Platte (2) mit einer Körperseite oder einer proximalen Oberfläche und einer distalen Oberfläche und enthaltend eine Schicht eines hautfreundlichen Haftmaterials und eine Stoma-Aufnahmeöffnung (5) für die Aufnahme eines Stomas des Patienten,
- eine im wesentlichen ringförmige Stirnplatten-Kopplungseinrichtung (6), die einen inneren (8) und einen äußeren (7) Umfang hat und dazu eingerichtet ist, fest oder lösbar mit einer entsprechenden Beutelkopplungseinrichtung (6) des Ostomiebeutels (6b) in Eingriff zu gelangen, wobei die Stirnplatten-Kopplungseinrichtung (6) an der flexiblen Platte (2) derart befestigt ist, dass die Ebene der Stirnplatten-Kopplungseinrichtung (6) im wesentlichen parallel zur Ebene der flexiblen Platte verläuft und im wesentlichen konzentrisch mit der Öffnung zur Aufnahme des Stomas ist, und
- ein Filter (17) zum Deodorieren eines Flatusgases,
**gekennzeichnet durch**
- ein Flatusgasentlüftungsabteil (16) in Gestalt eines Beutels oder einer Tasche, die aus einer ersten Folie (12) eines Kunststoffmaterials besteht und sich nach außen über den Außenumfang (7) hinaus erstreckt, wobei die erste Folie (12) mit einer Flatusgasauslassöffnung (18) versehen ist, und
- einen Flatusgasleitungsweg, der sich vom Innenumfang (8) in das Innere der Entlüftungstasche (16) und sowohl **durch** das Filter (17) als auch die Flatusgasauslassöffnung (18) erstreckt, wobei das Filter derart angeordnet ist, dass im wesentlichen das gesamte Flatusgas, das **durch** die Flatusgasöffnung (18) entlüftet wird, **durch** das Filter (17) strömt.

35. Stirnplatte nach Anspruch 34, bei der die Stirnplatten-Kopplungseinrichtung (6) und die Beutelkopplungseinrichtung (6a) wechselseitig zueinander passende Kopplungsringe sind, die dazu eingerichtet sind, miteinander lösbar in Eingriff zu gelangen.

36. Stirnplatte nach Anspruch 34, bei der die Stirnplatten-Kopplungseinrichtung (6) und die Beutelkopplungseinrichtung (6a) wechselseitig zueinander passende Oberflächen sind, die dazu eingerichtet sind, mit Hilfe eines Haftmittels (73) lösbar oder fest aneinander zu haften oder mit Hilfe einer Schweißnaht (74) fest aneinander zu haften.

37. Stirnplatte nach einem der Ansprüche 33 bis 36, bei der sich der Flatusgasleitungsweg zwischen der Stirnplatten-Kopplungseinrichtung (6) und der flexiblen Platte (2, 3, 4) erstreckt.

38. Stirnplatte nach einem der Ansprüche 33 bis 36, bei der sich der Flatusgasleitungsweg wenigstens teilweise durch eine Leitung (50) erstreckt, die durch einen Abschnitt der Stirnplatten-Kopplungseinrichtung (6) von einem Leitungseinlass zu einem Leitungsauslass verläuft.

39. Stirnplatte nach Anspruch 38, bei der ein Rand der Entlüftungstasche (16) an einer Körperseite oder der proximalen Oberfläche (53) der Stirnplatten-Kopplungseinrichtung (6) angebracht ist, die der flexiblen Platte zugewandt ist, und der gegenüberliegende Rand der Entlüftungstasche an einer distalen Oberfläche (54) der Stirnplatten-Kopplungseinrichtung (6) angebracht ist, die der flexiblen Platte abgewandt ist, wobei der Leitungsauslass zwischen der proximalen und der distalen Oberfläche derart angeordnet ist, dass Flatusgas, das aus der Leitung austritt, in die Entlüftungstasche (16) eintritt.

40. Stirnplatte nach einem der Ansprüche 36 bis 39, bei der sich die Tasche (16) entlang eines Teils der Ausdehnung des Außenumfangs (7) erstreckt.

41. Stirnplatte nach einem der Ansprüche 36 bis 39, bei der sich die Tasche (16) entlang der gesamten Ausdehnung des Außenumfangs (7) erstreckt.

42. Stirnplatte nach einem der Ansprüche 34 bis 41, bei der ein erster Rand eines ersten Folienstreifens (9) eines Kunststoffmaterials an dem Kopplungsring (6) angebracht ist und ein zweiter Rand des ersten Folienstreifens (9), gegenüberliegend dem ersten Rand, an der flexiblen Platte (2, 3, 4) innerhalb des Außenumfangs (7) angebracht ist, wobei wenigstens eine Flatusgaseinlassöffnung (19, 20) in dem ersten Folienstreifen vorgesehen ist.

43. Stirnplatte nach einem der Ansprüche 34 bis 42, bei der ein offenes Zellschaummaterial (25) in der Tasche stromaufwärts des Filters (17) derart angeordnet ist, dass im wesentlichen die gesamte Querschnittsfläche der Entlüftungstasche (16) mit dem Schaummaterial (25) an wenigstens einem Punkt gefüllt ist.

44. Stirnplatte nach einem der Ansprüche 34 bis 43, bei der eine Absperreinrichtung (65) benachbart des Innenumfangs (8) vorgesehen ist, die einen Eintritt stomaler Fluids und Flüssigkeiten in den Flatusgasleitungsweg behindert oder vermeidet.

45. Stirnplatte nach Anspruch 44, bei der die Absperreinrichtung (65) eine gasdurchlässige, flüssigkeitsundurchlässige Folie enthält, die derart angeordnet ist, dass die Folie den Einlass in den Flatusgasleitungsweg bedeckt.

46. Stirnplatte nach Anspruch 44 und 45, bei der die Folie am ersten Folienstreifen (9) in einem Bereich angebracht ist, der die Flatusgaseinlassöffnung (20) umgibt.

47. Stirnplatte nach Anspruch 45 und 42, bei der der erste Folienstreifen (9) aus der gasdurchlässigen, flüssigkeitsundurchlässigen Folie besteht.

48. Stirnplatte nach einem der Ansprüche 44 bis 47, bei der die Absperreinrichtung (65) einen zweiten Folienstreifen enthält, dessen einer Längsrand an der flexiblen Platte (2, 3, 4) innerhalb des Innenumfangs (8) entlang des gesamten Umfangs des Kopplungsringes (6) angebracht ist, wobei sich der gegenüberliegende Längsrand des zweiten Folienstreifens derart frei bewegen kann, dass ein ringförmiger Kranz, der sich von der flexiblen Platte (2, 3, 4) weg erstreckt, durch den zweiten Folienstreifen ausgebildet ist.

49. Stirnplatte nach einem der Ansprüche 44 bis 47, bei der die Absperreinrichtung (65) einen dritten Folienstreifen enthält, dessen einer Längsrand an dem Kopplungsring (6) entlang eines Teils seines gesamten Umfangs angebracht ist und sich vom Innenumfang (8) nach innen erstreckt, wobei sich der gegenüberliegende Längsrand des dritten Folienstreifens derart frei bewegen kann, dass eine Abdeckschürze, die sich im wesentlichen parallel zu der flexiblen Platte erstreckt, durch den dritten Folienstreifen ausgebildet ist.

50. Stirnplatte nach Anspruch 49, bei der sich der Einlass (20) in den Flatusgasleitungsweg an diesem Teil des Gesamtumfangs, vorzugsweise in der Nähe seiner Mitte befindet

51. Stirnplatte nach Anspruch 44, bei der die Absperreinrichtung (65) einen länglichen Körper eines gasdurchlässigen, vorzugsweise wasserabweisenden Materials, wie etwa einen offenen Zellschaum oder ein Vliesmaterial, enthält, wobei dieser Körper eine bogenförmige, vorzugsweise geschlossene kreisförmige und vorzugsweise ringförmige Gestalt hat und sich zwischen der Stirnplatten-Kopplungseinrichtung (6) und der Stoma-Aufnahmeöffnung (5) befindet.

52. Stirnplatte nach Anspruch 51, bei der der Körper an die distale Oberfläche der flexiblen Platte (2, 3, 4) im wesentlichen entlang der gesamten Länge des Körpers anschlägt.

53. Stirnplatte nach Anspruch 51 oder 52, bei der eine ringförmige Folie (67) an der proximalen Oberfläche des Körpers angebracht ist und wenigstens einen Teil, vorzugsweise jedoch im wesentlichen den gesamten Bereich, der distalen Oberfläche bedeckt, die sich zwischen dem Körper und der Stoma-Aufnahmeöffnung (5) befindet.

54. Stirnplatte nach Anspruch 53, bei der sich die ringförmige Folie (67) weg von dem Körper über den Umfang der Stoma-Aufnahmeöffnung (5) hinaus derart erstreckt, dass ein Stoma (69), das in der Stoma-Aufnahmeöffnung (5) aufgenommen ist, teilweise von der ringförmigen Folie in Gestalt einer Stulpe oder Manschette (68) für das Stoma bedeckt ist.

55. Stirnplatte nach einem der Ansprüche 34 bis 53, bei der ein Labyrinth in der Tasche (16) zwischen dem Einlass (20) in den Flatusgasleitungsweg und dem Filter (17) derart vorgesehen ist, dass der Flatusgasleitungsweg das Labyrinth enthält.

56. Stirnplatte nach Anspruch 55 und einem der Ansprüche 40 bis 54, bei der das Labyrinth einen vierten Folienstreifen (21) eines Kunststoffmaterials enthält, der dazu angeordnet und eingerichtet ist, die Tasche (16) in zwei Kammern (22 und 23) zu unterteilen, wobei eine Kammer (22) direkt mit dem Flatusgasleitungswegeinlass in Verbindung steht und die andere Kammer (23) das Filter enthält, wobei wenigstens eine Flatusgasströmungsöffnung (24) in dem vierten Folienstreifen vorgesehen ist.

57. Stirnplatte nach Anspruch 38, bei der Abstandshalteeinrichtungen (28, 29) wenigstens benachbart dem Leitungsauslass (50) derart vorgesehen sind, dass ein Zwischenraum zwischen den Folienwänden der Tasche geschaffen ist, damit Flatusgas durch die Flatusgasleitung (50) ungehindert durch eine Haftung zwischen den Folienwänden strömen kann.

58. Stirnplatte nach Anspruch 42, bei der die Abstandshalteeinrichtungen (28, 29) wenigstens benachbart der Flatusgaseinlassöffnung (19, 20) derart vorgesehen sind, dass ein Zwischenraum stromabwärts der Flatusgaseinlassöffnung (19, 20) gebildet ist, damit Flatusgas durch die Flatusgaseinlassöffnung (19, 20) ungehindert durch eine Haftung zwischen dem ersten Folienstreifen und einer Taschenwand oder der flexiblen Platte strömen kann.

59. Stirnplatte nach Anspruch 57 oder 58, bei der die Abstandshalteeinrichtungen (28, 29) einen Streifen eines Vliesmaterials enthalten.

60. Stirnplatte nach einem der vorhergehenden Ansprüche, bei der die proximale Oberfläche der flexiblen Platte (2, 3, 4) konvex ist, wobei ein relativ steifer konvexer Ring (76) an der distalen Oberfläche der flexiblen Platte angebracht ist.

61. Stirnplatte nach Anspruch 60, bei der Flatusgasleitungswege den konvexen Ring (76) durchlaufend vorgesehen sind.

62. Ostomievorrichtung zur Aufnahme menschlichen Stomaausflusses, enthaltend in Kombination eine selbstklebende Stirnplatte nach einem der vorhergehenden Ansprüche und einen Ostomiebeutel zur Aufnahme menschlichen Stomaausflusses, der lösbar oder fest an der Stirnplatte angebracht ist.

63. Ostomievorrichtung nach Anspruch 62, bei der der Ostomiebeutel (6b) einen einzigen Beutel eines gas- und flüssigkeitsundurchlässigen Materials enthält.

64. Ostomievorrichtung nach Anspruch 62, bei der der Ostomiebeutel zwei Beutel enthält, einen Außenbeutel (61) eines gas- und flüssigkeitsundurchlässigen Materials und einen Innenbeutel (63) eines flexiblen Materials, der in dem Außenbeutel (61) enthalten ist.

65. Ostomievorrichtung nach Anspruch 63, ebenso abhängig von einem der Ansprüche 23 bis 26 oder 51 bis 54, bei der der längliche Körper (90) an dem Ostomiebeutel (6b) und oder der Beutelkopplungseinrichtung (6a) derart angebracht ist, dass ein Entfernen des Ostomiebeutels (6b) von der Stirnplatte (1) des Entfernen des länglichen Körpers von der Stirnplatte bedingt.

66. Ostomievorrichtung nach Anspruch 64 ebenso abhängig von einem der Ansprüche 23 bis 26 oder 51 bis 54, bei der der längliche Körper (90) an dem inneren Ostomiebeutel (63) derart angebracht ist, dass ein Entfernen des inneren Ostomiebeutels (63) von der Stirnplatte (1) das Entfernen des länglichen Körpers (90) von der Stirnplatte (1) bedingt.

## Revendications

1. Plaque avant adhésive (1) pour fixer un sac d'ostomie (6b) à la peau d'un patient subissant une ostomie, ladite plaque avant (1) comprenant :
- une plaque flexible (2) avec un côté de corps ou une surface proximale et une surface distale et comprenant une couche d'un matériau adhésif non nocif pour la peau et une ouverture recevant un stoma (5) pour recevoir un stoma dudit patient,
- un système de couplage de plaque avant généralement annulaires (6) ayant une périphérie interne (8) et externe (7) et adapté pour mettre en prise de manière fixe ou amovible un système de couplage de sac correspondant (6) dudit sac de stomie (6b), ledit système de couplage de plaque avant (6) étant fixé à ladite plaque flexible (2), le plan dudit système de couplage de plaque avant (6) étant généralement parallèle au plan de ladite plaque et généralement concentrique avec ladite ouverture pour recevoir ledit stoma, et
- un filtre (17) pour désodoriser les gaz intestinaux.
**caractérisée par**
- un ou plusieurs passages de gaz intestinaux s'étendant entre le système de couplage de plaque avant (6) et ladite plaque flexible (2) depuis ladite périphérie interne (8) vers ladite périphérie externe (7) et à travers ledit filtre (17).

2. Plaque avant selon la revendication 1, dans laquelle ledit système de couplage de plaque avant (6) et ledit système de couplage de sac (6a) sont des anneaux de couplage réciproquement adaptables, adaptés pour se mettre en prise de manière amovible l'un avec l'autre.

3. Plaque avant selon la revendication 1, dans laquelle ledit système de couplage de plaque avant (6) et ledit système de couplage de sac (6a) sont des surfaces s'adaptant réciproquement, adaptées pour adhérer de manière fixe ou amovible l'une à l'autre au moyen d'un adhésif (73) ou pour adhérer de manière fixe l'une à l'autre au moyen d'un joint thermo-soudé (74).

4. Plaque avant selon la revendication 3, dans laquelle une ou plusieurs desdites surfaces s'adaptant réciproquement sont dotées d'un anneau (71, 72) en un matériau relativement rigide.

5. Plaque avant selon l'une quelconque des revendications précédentes et comprenant en outre un compartiment (16) ayant une partie extérieure (16b) située à l'extérieur de ladite périphérie extérieure (7) et une partie intérieure (16a) située entre lesdits systèmes de couplage de la plaque avant (6) et ladite plaque flexible (2), une sortie de gaz intestinaux (18) étant ménagée dans une paroi (12) de ladite partie extérieure, ledit compartiment formant une partie dudit passage de gaz intestinaux, ledit filtre (17) étant situé dans ledit compartiment, de sorte que ladite sortie de gaz intestinaux soit obstruée par ledit filtre (17).

6. Plaque avant selon la revendication 5, dans laquelle ladite partie extérieure (16b) dudit compartiment (16) est définie par une première bande de film (9) en matière plastique, de préférence adaptée pour définir une poche.

7. Plaque avant selon la revendication 6, dans laquelle ladite première bande de film (9) a un premier bord fixé audit système de couplage de plaque avant (6) et un second bord opposé audit premier bord, fixé à ladite plaque flexible (2) de façon à permettre audit passage de gaz intestinaux de s'étendre entre lesdits premier et second bords.

8. Plaque avant selon la revendication 7, dans laquelle ladite première bande de film (9) est composée d'une seconde et d'une troisième bandes de film en matière plastique, ladite seconde et ladite troisième bandes de film comprenant ledit premier bord et ledit second bord, respectivement, et lesdites seconde et troisième bandes de film étant fixées l'une à l'autre, le long de leurs bords opposés auxdits premier et second bords, respectivement.

9. Plaque avant selon la revendication 6, dans laquelle ladite première bande de film (9) a un premier bord fixé audit anneau de couplage et un second bord opposé audit premier bord, fixé à ladite plaque flexible à l'extérieur de ladite périphérie extérieure.

10. Plaque avant selon l'une quelconque des revendications 5-9, dans laquelle ladite partie extérieure (16b) dudit compartiment (16) s'étend le long de la circonférence de ladite périphérie extérieure (7).

11. Plaque avant selon l'une quelconque des revendications 5-9, dans laquelle ladite partie extérieure (16b) dudit compartiment (16) s'étend le long de toute la circonférence de ladite périphérie extérieure (7).

12. Plaque avant selon l'une quelconque des revendications 3-11, dans laquelle un premier bord d'une quatrième bande de film (12) en matière plastique est fixé audit système de couplage de plaque avant (6) et un second bord de ladite quatrième bande de film (12) opposé audit premier bord est fixé à ladite plaque flexible (2) à l'intérieur de ladite périphérie extérieure, au moins une ouverture d'entrée de gaz intestinaux (19, 20) étant ménagée dans ladite quatrième bande de film (12) constituant une entrée de gaz intestinaux vers un passage correspondant desdits au moins un passage de gaz intestinaux.

13. Plaque avant selon la revendication 12, dans laquelle ladite quatrième bande de film (12) est d'un seul tenant avec ladite première bande de film (9).

14. Plaque avant selon la revendication 12 ou 13, dans laquelle ladite ouverture d'entrée de gaz intestinaux (19, 19a, 19b, 20, 24) est éloignée de ladite ouverture de sortie de gaz (18).

15. Plaque avant selon la revendication 14, dans laquelle un matériau en mousse à cellules ouvertes (25) est disposé dans ledit compartiment (16) entre ladite ouverture d'entrée de gaz intestinaux (19, 20) et ladite sortie de gaz intestinaux (18) de sorte que sensiblement toute la section transversale du compartiment (16) soit remplie dudit matériau de mousse au moins en un endroit entre ladite ouverture d'entrée de gaz intestinaux (19, 20) et ladite sortie de gaz intestinaux (18).

16. Plaque avant selon l'une quelconque des revendications précédentes, dans laquelle des systèmes d'obstruction (65) pour arrêter ou empêcher l'entrée des fluides et liquides de stomie dans ledit passage de gaz intestinaux sont fournis près de ladite périphérie interne (8).

17. Plaque avant selon la revendication 16, dans laquelle lesdits systèmes d'obstruction (65) comprennent un film perméable au gaz et imperméable au liquide disposé de sorte que ledit film recouvre l'entrée dudit passage de gaz intestinaux.

18. Plaque avant selon la revendication 17, et l'une quelconque des revendications 12-16, dans laquelle ledit film est fixé à ladite quatrième bande de film (12) dans une zone entourant ladite ouverture d'entrée de gaz intestinaux (20).

19. Plaque avant selon la revendication 17 et l'une quelconque des revendications 12-16, dans laquelle ladite quatrième bande de film (12) est constituée dudit film imperméable au liquide et perméable au gaz.

20. Plaque avant selon l'une quelconque des revendications 16-19, dans laquelle ledit système d'obstruction (65) comprend une cinquième bande de film avec un bord longitudinal fixé à la plaque flexible (2, 3, 4), à l'intérieur de ladite périphérie (8) le long de toute la circonférence dudit anneau de couplage (6), le bord longitudinal opposé de ladite cinquième bande de film étant libre de bouger, de sorte qu'un collier annulaire s'étendant loin de ladite plaque flexible soit formé par ladite cinquième bande de film.

21. Plaque avant selon l'une quelconque des revendications 16-20, dans laquelle le système d'obstruction (65) comprend une sixième bande de film avec un bord longitudinal fixé audit anneau de couplage (6), le long d'une partie de la circonférence totale de celui-ci, et s'étendant vers l'intérieur depuis ladite périphérie interne (8), le bord longitudinal opposé de ladite sixième bande de film étant libre de bouger de sorte qu'un rideau s'étendant généralement parallèlement à la plaque flexible soit formé par ladite sixième bande de film.

22. Plaque avant selon la revendication 21, dans laquelle ladite entrée dudit passage de gaz intestinaux est située dans ladite partie de la circonférence totale, de préférence près de son centre.

23. Plaque avant selon la revendication 16, dans laquelle ledit système d'obstruction (65) comprend un corps allongé d'un matériau perméable au gaz, de préférence hydrophobe, comme une mousse à cellules ouvertes ou un matériau non tissé, ledit corps ayant une forme arquée, de préférence en boucle fermée et de préférence une forme annulaire et étant situé entre ledit système de couplage de plaque avant (6) et ladite ouverture recevant le stoma (5).

24. Plaque avant selon la revendication 23, dans laquelle ledit corps vient en butée contre ladite surface distale de ladite plaque flexible (2, 3, 4), le long sensiblement de toute la longueur dudit corps.

25. Plaque avant selon la revendication 23 ou 24, dans laquelle un film annulaire (67) est fixé à la surface proximale dudit corps et recouvre au moins une partie, de préférence sensiblement toute la zone de ladite surface distale située entre ledit corps et ladite ouverture recevant le stoma (5).

26. Plaque avant selon la revendication 25, dans laquelle ledit film annulaire (67) s'étend loin dudit corps au delà de la périphérie de ladite ouverture recevant le stoma (5), de sorte qu'un stoma (69) reçu dans ladite ouverture recevant le stoma (5) soit partiellement recouvert par ledit film annulaire sous la forme d'une gaine ou d'une coiffe (68) pour ledit stoma.

27. Plaque avant selon l'une quelconque des revendications 5-26, dans laquelle un labyrinthe est ménagé dans ledit compartiment (16) entre l'entrée (19, 20) dudit passage de gaz intestinaux et ledit filtre (17), de sorte que ledit passage de gaz intestinaux comprenne ledit labyrinthe.

28. Plaque avant selon la revendication 27 et l'une quelconque des revendications 12-26, dans laquelle ledit labyrinthe comprend une septième bande de film (21) en matière plastique ayant un premier bord fixé à ladite plaque flexible (2, 3, 4) avec ledit second bord de ladite quatrième bande de film (12), ladite septième bande de film (21) ayant une seconde bande de bord opposée audit premier bord correspondant, fixée à ladite première bande de film (9), de sorte que le compartiment défini entre ladite première bande de film (9) et ladite quatrième bande de film (12) soit subdivisé en deux chambres (22, 23), au moins une ouverture de flux de gaz intestinaux (24) étant ménagée dans ladite septième bande de film.

29. Plaque avant selon l'une quelconque des revendications 12-26, dans laquelle ledit système d'espacement (28, 29) est placé entre ladite quatrième bande de film (12) et ladite première bande de film (9), de manière au moins adjacente à ladite ouverture d'entrée de gaz intestinaux (19, 20), de sorte qu'un espace soit ménagé entre ladite première (9) et ladite quatrième (12) bandes de film pour permettre aux gaz intestinaux de s'écouler à travers ladite ouverture d'entrée de gaz intestinaux (91, 20), sans être gênés par une adhérence quelconque entre ladite première (9) et ladite quatrième (12) bandes de film.

30. Plaque avant selon l'une quelconque des revendications 28, dans laquelle ledit système d'espacement (28, 29) est placé entre ladite quatrième bande de film (12) et ladite septième bande de film (21), de manière adjacente à ladite ouverture d'entrée de gaz intestinaux (19, 20) de sorte qu'un espace soit ménagé entre ladite première (9) et ladite septième (21) bandes de film pour permettre aux gaz intestinaux de s'écouler à travers ladite ouverture d'entrée du gaz intestinaux (19, 20) sans être gênés par une adhérence quelconque entre ladite septième (21) et ladite quatrième (12) bandes de film.

31. Plaque avant selon la revendication 29 ou 30, dans laquelle ledit système d'espacement (28, 29) comprend une bande de matériau non tissé.

32. Plaque avant selon l'une quelconque des revendications 1-4, dans laquelle ledit filtre (42) est annulaire et est disposé entre ledit système de couplage de plaque avant (6) et ladite plaque flexible (2, 3, 4).

33. Plaque avant selon la revendication 32, dans laquelle ledit anneau de couplage (6) est fixé à ladite plaque flexible (4) au moyen dudit filtre annulaire (42).

34. Plaque avant adhésive (1) pour fixer un sac de stomie (6b) à la peau d'un patient subissant une stomie, ladite plaque avant comprenant :
- une plaque flexible (2) avec un côté de corps ou une surface proximale et une surface distale et comprenant une couche d'un matériau adhésif non nocif pour la peau, et une ouverture recevant un stoma (5) pour recevoir un stoma dudit patient,
- un système de couplage de plaque avant généralement annulaire (6) ayant une périphérie interne (8) et externe (7) et adapté pour mettre en prise de manière fixe ou amovible un système de couplage de sac correspondant (6) dudit sac de stomie (6b), ledit système de couplage de plaque avant (6) étant fixé à ladite plaque flexible (2), le plan dudit système de couplage de plaque avant (6) étant généralement parallèle au plan de ladite plaque flexible (2) et généralement concentrique avec ladite ouverture (5) pour recevoir ledit stoma, et
- un filtre (17) pour désodoriser les gaz intestinaux,
**caractérisée par**
- un compartiment d'aération de gaz intestinaux (16) sous la forme d'une poche ou d'un sac réalisé en un premier film (12) en matière plastique et s'étendant vers l'extérieur au delà de ladite périphérie extérieure (7), ledit premier film (12) étant doté d'une ouverture de sortie de gaz intestinaux (18) et
- un passage de gaz intestinaux s'étendant depuis ladite périphérie interne (8) vers l'intérieur de ladite poche de ventilation (16) et à travers ledit filtre (17) et ladite ouverture de sortie de gaz intestinaux (18), ledit filtre étant disposé de sorte que sensiblement tous les gaz intestinaux ventilés à travers ladite ouverture de gaz intestinaux (18) s'écoulent à travers ledit filtre (17).

35. Plaque avant selon la revendication 34, dans laquelle ledit système de couplage de plaque avant (6) et ledit système de couplage de sac (6a) sont des anneaux de couplage s'adaptant réciproquement, adaptés pour se mettre en prise réciproquement l'un avec l'autre.

36. Plaque avant selon la revendication 34, dans laquelle ledit système de couplage de plaque avant (6) et ledit système de couplage de sac (6a) sont des surfaces s'adaptant réciproquement, adaptées pour adhérer de manière amovible ou fixe l'une à l'autre, au moyen d'un adhésif (73) ou adhérer de manière fixe l'une à l'autre au moyen d'un joint thermo-soudé (74).

37. Plaque avant selon l'une quelconque des revendications 33-36, dans laquelle ledit passage de gaz intestinaux s'étend entre ledit système de couplage de plaque avant (6) et ladite plaque flexible (2, 3, 4).

38. Plaque avant selon l'une quelconque des revendications 33-36, dans laquelle ledit passage de gaz intestinaux s'étend au moins partiellement à travers un conduit (50) s'étendant à travers une partie dudit système de couplage de plaque avant (6) depuis une entrée de conduit vers une sortie de conduit.

39. Plaque avant selon la revendication 38, dans laquelle un bord de ladite poche d'aération (16) est fixé à un côté du corps ou surface proximale (53) dudit système de couplage de plaque avant (6) faisant face à ladite plaque flexible et le bord opposé de ladite poche d'aération est fixé à une surface distale (54) dudit système de couplage de plaque avant (6) orienté loin de ladite plaque flexible, ladite sortie de conduit étant située entre lesdites surfaces proximale et distale, de sorte que les gaz intestinaux sortant dudit conduit entrent dans ladite poche d'aération (16).

40. Plaque avant selon l'une quelconque des revendications 36-39, dans laquelle ladite poche (16) s'étend le long d'une partie de la circonférence de ladite périphérie extérieure (7).

41. Plaque avant selon l'une quelconque des revendications 36-39, dans laquelle ladite poche (16) s'étend le long de toute la circonférence de ladite périphérie extérieure (7).

42. Plaque avant selon l'une quelconque des revendications 34-41, dans laquelle un premier bord d'une première bande de film (9) en matière plastique est fixé audit anneau de couplage (6) et un second bord de ladite première bande de film (9) opposé audit premier bord est fixé à ladite plaque flexible (2, 3, 4) à l'intérieur de ladite périphérie extérieure (7), au moins une ouverture d'entrée de gaz intestinaux (19, 20) étant ménagée dans ladite première bande de film.

43. Plaque avant selon l'une quelconque des revendications 34-42, dans laquelle le matériau en mousse à cellules ouvertes (25) est disposé dans ladite poche en amont dudit filtre (17), de sorte que sensiblement toute la superficie transversale de ladite poche d'aération (16) soit remplie dudit matériau de mousse (25) au moins en un point.

44. Plaque avant selon l'une quelconque des revendications 34-43, dans laquelle des systèmes d'obstruction (65) pour gêner ou empêcher les fluides et liquides de stomie d'entrer dans ledit passage de gaz intestinaux sont fournis près de ladite périphérie interne (8).

45. Plaque avant selon la revendication 44, dans laquelle ledit système d'obstruction (65) comprend un film imperméable au liquide, perméable au gaz, disposé de sorte que ledit film recouvre l'entrée audit passage de gaz intestinaux.

46. Plaque avant selon les revendications 44 et 45, dans laquelle ledit film est fixé à ladite bande de film (9) dans une zone entourant ladite ouverture d'entrée de gaz intestinaux (20).

47. Plaque avant selon la revendication 45 et la revendication 42, dans laquelle ladite première bande de film (9) est constituée par ledit film imperméable au liquide, perméable au gaz.

48. Plaque avant selon l'une quelconque des revendications 44-47, dans laquelle ledit système d'obstruction (65) comprend une seconde bande de film avec un bord longitudinal fixé à la plaque flexible (2, 3, 4) à l'intérieur de ladite périphérie interne (8), le long de la circonférence dudit anneau de couplage (6), le bord longitudinal opposé de ladite seconde bande de film étant libre de bouger, de sorte qu'un collier annulaire s'étendant loin de ladite plaque flexible (2, 3, 4) soit formé par ladite seconde bande de film.

49. Plaque avant selon l'une quelconque des revendications 44-47, dans laquelle le système d'obstruction (65) comprend une troisième bande de film avec un bord longitudinal fixé audit anneau de couplage (6), le long d'une partie de la circonférence totale correspondante et s'étendant vers l'intérieur depuis ladite périphérie interne (8), le bord longitudinal opposé de ladite troisième bande de film étant libre de bouger, de sorte qu'un rideau s'étendant généralement parallèlement à la plaque flexible soit formé par ladite troisième bande de film.

50. Plaque avant selon la revendication 49, dans laquelle ladite entrée (20) dudit passage de gaz intestinaux est située sur ladite partie de la circonférence totale, de préférence près de son centre.

51. Plaque avant selon la revendication 44, dans laquelle ledit système d'obstruction (65) comprend un corps allongé en matériau perméable au gaz, de préférence hydrophobe comme une mousse à cellules ouvertes, ou en matériau non tissé, ledit corps ayant une forme arquée, et de préférence en boucle fermée, et étant situé entre ledit système de couplage de plaque avant (6) et ladite ouverture recevant un stoma (5).

52. Plaque avant selon la revendication 51, dans laquelle ledit corps vient en butée contre ladite surface distale de ladite plaque flexible (2, 3, 4) sensiblement le long de toute la longueur dudit corps.

53. Plaque avant selon la revendication 51 ou 52, dans laquelle un film annulaire (67) est fixé à la surface proximale dudit corps et recouvre au moins une partie, de préférence sensiblement toute la zone de ladite surface distale située entre ledit corps et ladite ouverture recevant le stoma (5).

54. Plaque avant selon la revendication 53, dans laquelle ledit film annulaire (67) s'étend loin dudit corps au delà de la périphérie de ladite ouverture recevant le stoma (5), de sorte qu'un stoma (69) reçu dans ladite ouverture recevant le stoma (5) soit partiellement recouvert par ledit film annulaire (67) sous la forme d'une gaine ou coiffe (68) pour ledit stoma.

55. Plaque avant selon l'une quelconque des revendications 34-53, dans laquelle un labyrinthe est ménagé dans ladite poche (16) entre l'entrée (20) dudit passage de gaz intestinaux et ledit filtre (17), de sorte que ledit passage de gaz intestinaux comprenne ledit labyrinthe.

56. Plaque avant selon la revendication 55 et l'une quelconque des revendications 40-54, dans laquelle ledit labyrinthe comprend une quatrième bande de film (21) en matière plastique, disposée et adaptée pour subdiviser ladite poche (16) en deux chambres (22 et 23), une chambre (22) communiquant directement avec ladite entrée de passage de gaz intestinaux et l'autre chambre (23) contenant ledit filtre, au moins une ouverture d'écoulement de gaz intestinaux (24) étant ménagée dans ladite quatrième bande de film.

57. Plaque avant selon la revendication 38, dans laquelle un système d'espacement (28, 29) est fourni au moins de manière adjacente à ladite sortie de conduit (50) de sorte qu'un espace soit ménagé entre les parois du film de ladite poche pour permettre aux gaz intestinaux de s'écouler à travers ledit conduit de gaz intestinaux (50) sans être gênés par une adhérence quelconque entre lesdites parois de film.

58. Plaque avant selon la revendication 42, dans laquelle un système d'espacement (28, 29) est ménagé au moins près de ladite ouverture d'entrée de gaz intestinaux (19, 20), de sorte qu'un espace soit ménagé en aval de ladite ouverture d'entrée de gaz intestinaux (19, 20), pour permettre aux gaz intestinaux de s'écouler à travers ladite ouverture d'entrée de gaz intestinaux (19, 20) sans être gênés par une adhérence quelconque entre ladite première bande de film et une paroi de poche ou la plaque flexible.

59. Plaque avant selon la revendication 57 ou 58, dans laquelle le système d'espacement (28, 29) comprend une bande de matériau non tissé.

60. Plaque avant selon l'une quelconque des revendications précédentes, dans laquelle ladite surface proximale de ladite plaque flexible (2, 3, 4) est convexe, un anneau convexe relativement rigide (76) étant fixé à ladite surface distale de ladite plaque flexible.

61. Plaque avant selon la revendication 60, dans laquelle des passages de gaz intestinaux sont fournis à travers ledit anneau convexe (76).

62. Appareil d'ostomie pour recevoir une décharge de stomie humaine comprenant en combinaison une plaque avant adhésive selon l'une quelconque des revendications précédentes et un sac d'ostomie pour recevoir une décharge de stomie humaine, fixé de manière amovible ou fixe à ladite plaque avant.

63. Appareil d'ostomie selon la revendication 62, dans lequel ledit sac d'ostomie (6b) comprend un sac unique en matériau imperméable au liquide et au gaz.

64. Appareil d'ostomie selon la revendication 62, dans lequel ledit sac d'ostomie comprend deux sacs, un sac extérieur (61) en matériau imperméable au gaz et au liquide et un sac intérieur (63) en matériau flexible et contenu dans ledit sac extérieur (61).

65. Appareil d'ostomie selon la revendication 63, dépendant de l'une quelconque des revendications 23-26 ou 51-35 54, dans lequel ledit corps allongé (90) est fixé audit sac d'ostomie (6b) et ou audit système de couplage de sac (6a), de sorte que l'enlèvement dudit sac d'ostomie (6b) de ladite plaque avant (1) provoque l'enlèvement dudit corps allongé de ladite plaque avant.

66. Appareil d'ostomie selon la revendication 64, dépendant de l'une quelconque des revendications 23-26 ou 51-54, dans lequel ledit corps allongé (90) est fixé audit sac d'ostomie interne (63), de sorte que l'enlèvement dudit sac d'ostomie interne (63) de ladite plaque avant (1) provoque l'enlèvement dudit corps allongé (90) de chaque plaque avant (1).
